# EUROPEAN PATENT APPLICATION

(11) **EP 4 141 877 A1**
(43) Date of publication of application: **01.03.2023**
(21) Application number: 21194188.5
(22) Date of filing: 31.08.2021
(51) Int. Cl.: G16C 20/30, A61K 9/00, A61K 8/64

(54) **METHOD FOR IDENTIFYING A SOLVENT FOR A MATRIKINE**

(71) Applicant: The Boots Company plc, Nottingham NG90 1BS (GB)
(72) Inventor: JOHNSON, Mark, Nottingham, NG15 8DQ (GB); ELMS, Beverley Jane, Nottingham, NG13 OFH (GB); TOMLINSON, Paul James, Nottingham, DE22 1EX (GB)
(74) Representative: Teasdale, Andrew James

(57) **Abstract**

Methods of choosing a solvent for a matrikine, methods of creating a matrikine composition, compositions (e.g., cosmetic compositions) made by the methods, and uses thereof are provided.

## Description

### Technical Field

The present invention relates to a method of identifying a solvent for a peptide composition. The invention also extends to a method of creating a composition comprising a matrikine, composition obtained or obtainable by the method, and a method of delivering the composition to the skin of a subject.

### Background to the invention

Peptides have been used in cosmetic compositions and products for several years to stimulate peptide upregulation, skin repair and regeneration. The most commonly used peptide is the combination of palmitoyl oligopeptide (Pal-GHK), which acts as a messenger peptide for collagen renewal and consists of a sequence derived from collagen I, and palmitoyl tetrapeptide-7 (Pal-GQPR), which reduces the production of the inflammatory cytokine, interleukin-6 (IL-6), and inhibits extracellular matrix (ECM) degradation. This said peptide combination is available from Sederma under the trade name Matrixyl.

However, peptides have limited solubility in the skin due to their hydrophilic nature. In order to address this problem, peptides have been modified to make them more lipophilic. For example, by covalently attaching a lipophilic group, such as a fatty acid (e.g., palmitic acid), to the peptide. Such modified peptides are better absorbed by the skin (compared to the non-modified peptides) because of lipophilic and/or hydrophobic nature of skin.

Whilst natural and synthetic peptides have been in use for a number of years there remains a need to identify new and improved ways of delivering biologically active peptides into, onto and under the skin.

### Summary of the invention

Thus, according to one aspect of the invention, there is provided a method of choosing a solvent for a matrikine, the method comprising:
(a) identifying the Hansen Solubility Parameter (HSP) sphere of
   skin,
   the matrikine, and
   two or more test solvents for the matrikine; and
(b) choosing at least one of the two or more test solvents based on
   the proximity of its HSP sphere to the HSP sphere of skin, and/or
   the proximity of its HSP sphere to the HSP sphere of the matrikine,
   wherein the matrikine has an amino acid sequence selected from the group consisting of:
   (i) GPXG (SEQ ID No. 1), (ii) LSXX (SEQ ID No. 2), (iii) XXGD (SEQ ID No. 3), (iv) QTAV (SEQ ID No. 4), (v) GHK (SEQ ID No. 5), and (vi) GQPR (SEQ ID No. 6),

   wherein for (i) G denotes the amino acid glycine and P denotes the amino acid proline (as per the internationally recognised single letter code for amino acids), and X denotes an amino acid selected from the group consisting of Lysine (K), Glutamic acid (E) and Serine (S) and mixtures thereof,
   wherein for (ii) L denotes the amino acid Leucine and S denotes the amino acid Serine (as per the internationally recognised single letter code for amino acids), and X denotes an amino acid selected from the group consisting of Valine (V), Aspartic acid (D), Proline (P), Glycine (G) and mixtures thereof,
   wherein for (iii) G denotes the amino acid Glycine and D denotes the amino acid Aspartic acid (as per the internationally recognised single letter code for amino acids), and X denotes an amino acid selected from the group consisting of Glutamic acid (E), Lysine (K), Leucine (L), Alanine (A), Isoleucine (I), Arginine (R) and mixtures thereof,
   wherein for (iv) Q denotes the amino acid Glutamine, T denotes the amino acid Threonine, A denotes the amino acid Alanine, and V denotes the amino acid Valine,
   wherein for (v), G denotes the amino acid Glycine, H denotes Histidine, and K denotes the amino acid Lysine (as per the internationally recognised single letter code for amino acids), and
   wherein for (vi), G denotes the amino acid Glycine, Q denotes the amino acid Glutamine, P denotes the amino acid Proline, and R denotes the amino acid Arginine (as per the internationally recognised single letter code for amino acids).

Advantageously, the method according to the invention can be used to identify a solvent, for a matrikine, which can be used to create a composition (e.g., a cosmetic composition) that improves the delivery of the matrikine to the skin and/or improves the stability of the matrikine within the composition.

According to another aspect of the invention, there is provided a method of creating a composition comprising a matrikine. The method may comprise choosing a solvent for a matrikine using the method according to the invention; and dissolving the matrikine in the chosen solvent(s).

According to another aspect of the invention, there is provided a composition obtained or obtainable by the method according to the invention.

According to another aspect of the invention, there is provided a method of delivering a composition of the invention to the skin of a subject. The method may comprise contacting a composition according to the invention with the skin of said subject.

### Detailed description of the invention

The present invention is drawn to a method of identifying a solvent for a composition (e.g., a cosmetic composition) comprising a matrikine. The test solvent may be chosen based on the proximity of its HSP sphere to that of skin and/or the matrikine. The solvent may be chosen because it improves the stability of the matrikine within the composition and/or improves the delivery of the matrikine to skin.

### Hansen Solubility Parameter (HSP)

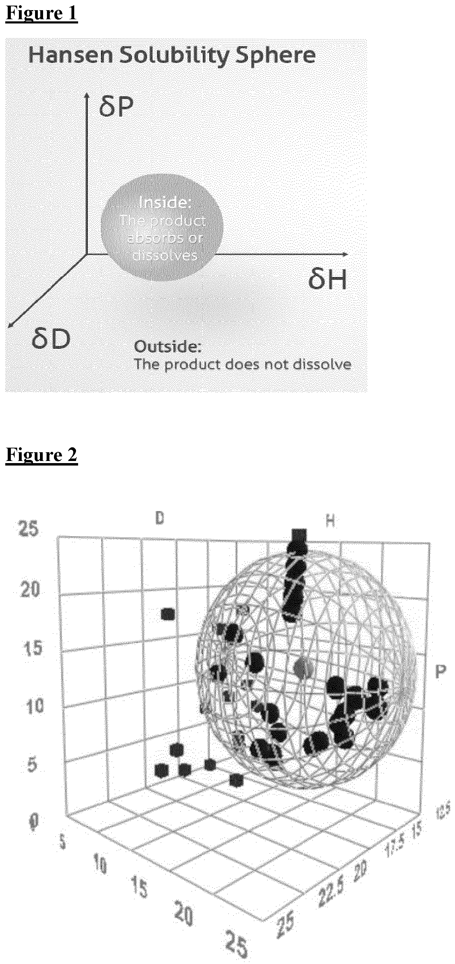

HSP refers to three different parameters of a molecule or compound, i.e., δD = Dispersion forces (Van der Waals), δP = Polarity, and δH = Hydrogen bonding (see Figure 1). Many molecules and compounds have HSP values, which (once determined) can be plotted in a HSP space (see Figure 2). Knowledge of these parameters for a given solute (e.g., a matrikine or skin) and solvent enables one to determine if the solute will dissolve in the solvent. A solvent, with similar HSP values to a solute, is likely to dissolve the solute. In other words, the more similar the HSP values of a solute are to those of a solvent, the more likely that solute will dissolve in the solvent.

### HSP sphere

The HSP values of a solute and a solvent do not need to be identical for complete solubility to occur. The solvents for a given a solute tend to have HSP values that are close in 3D proximity to the HSP values of the solute. Likewise, molecules that are incapable of dissolving a given solute have HSP values that are more distal from those of the solute. Thus, by testing the ability of several different solvents, each with known HSP values, to dissolve given a solute, a boundary (i.e., a HSP sphere) can be generated for the solute with the HSP values of the solute being in the centre of sphere (see Figure 2). Thus, solvents with HSP values within the sphere can be used to dissolve the solute, whereas potential solvents with HSP values outside the sphere cannot be used to dissolve the solute. The diameter/radius of a sphere varies from solute to solute.

### Step (a) - Identifying the HSP sphere

Identifying the HSP sphere according to the invention may comprise referring to reference HSP values, e.g., for skin and/or the two or more test solvents. Reference HSP values can be obtained by determining their chemical structure of the relevant chemical using Simplified molecular-input line-entry system (SMILES) notification, and then using the software, HSPiP (https://www.hansen-solubiliy.com/downloads.php; and/or the software, Formulating For Efficacy^{™} (https://www.jwsolutionssoftware.com/). Reference HSP values can also be obtained from various websites online, including, for example, www.specialchem.com.

Identifying the HSP sphere (for one or more of members selected from the group consisting of skin, the matrikine and the two or more test solvents) may comprise using one or more different reference solvents, i.e., known solvents with known HSP values. Identifying the HSP sphere may comprise determining the HSP values using one or more, two or more, three or more, four or more, five or more, six or more, seven or more, eight or more, nine or more, 10 or more, 11 or more, 12 or more 13 or more, 14 or more, 15 or more, 16 or more, 17 or more, 18 or more, 19 or more, 20 or more, 25 or more, 30 or more, 35 or more, 40 or more, 45 or more, or 50 or more different reference solvents.

Identifying the HSP sphere may comprise ranking each reference solvents based on their ability to dissolve skin, the matrikine and/or a test solvent by using the qualitative criteria of a solubility rank.

The solubility rank may use the following qualitative criteria:

| **Solubility Rank** | **Precipitate** | **Turbidity** |
|---|---|---|
| 1 | None | None |
| 2 | None | Weak |
| 3 | None | Strong |
| 4 | Trace | Strong |
| 5 | Moderate | Weak |
| 6 | Complete | None |

Reference solvents that are ranked 1 completely dissolve skin, the matrikine or a test solvent and thus fall within the relevant HSP sphere. Reference solvents that are ranked 2, 3, 4 or 5 may, or may not fall within the HSP sphere of skin, the HSP sphere of the matrikine or the HSP sphere of a test solvent. Reference solvents that are ranked 6 do not dissolve skin, the matrikine or a test solvent and thus fall outside the relevant HSP sphere.

Reference solvents with a solubility rank of 4, 3, 2 or 1 may be used to identify the HSP sphere of skin, the HSP sphere of the matrikine, or the HSP sphere of a test solvent. Preferably, reference solvents with a solubility rank of 3, 2 or 1 are used to identify the HSP sphere of skin, the HSP sphere of the matrikine, or the HSP sphere of a test solvent. More preferably, reference solvents with a solubility rank of 2 or 1 are used to identify the HSP sphere of skin, the HSP sphere of the matrikine, or the HSP sphere of a test solvent. Most preferably, reference solvents with a solubility rank of 1 are used to identify the HSP sphere of skin, the HSP sphere of the matrikine, or the HSP sphere of a test solvent.

The skilled person will appreciate that the more reference solvents that are used to determine the HSP sphere of the skin, the HSP sphere of the matrikine, and the HSP sphere of the two or more test solvents, the more accurate the identification of the HSP sphere will be. The skilled person will also appreciate that the higher the solubility rank of the reference solvents that are used to determine the HSP sphere of the skin, the HSP sphere of the matrikine, and the HSP sphere of the two or more test solvents, the more accurate the identification of the HSP sphere will be.

Thus, identifying the HSP sphere may comprise using one or more, two or more, three or more, four or more, five or more, six or more, seven or more, eight or more, nine or more, 10 or more, 11 or more, 12 or more 13 or more, 14 or more, 15 or more, 16 or more, 17 or more, 18 or more, 19 or more, 20 or more, 25 or more, 30 or more, 35 or more, 40 or more, 45 or more, or 50 or more different reference solvents with a solubility rank of 3, 2 or 1.

Preferably, identifying the HSP sphere may comprise using one or more, two or more, three or more, four or more, five or more, six or more, seven or more, eight or more, nine or more, 10 or more, 11 or more, 12 or more 13 or more, 14 or more, 15 or more, 16 or more, 17 or more, 18 or more, 19 or more, 20 or more, 25 or more, 30 or more, 35 or more, 40 or more, 45 or more, or 50 or more different reference solvents with a solubility rank of 2 or 1.

Most preferably, identifying the HSP sphere may comprise using one or more, two or more, three or more, four or more, five or more, six or more, seven or more, eight or more, nine or more, 10 or more, 11 or more, 12 or more 13 or more, 14 or more, 15 or more, 16 or more, 17 or more, 18 or more, 19 or more, 20 or more, 25 or more, 30 or more, 35 or more, 40 or more, 45 or more, or 50 or more different reference solvents with a solubility rank of 1.

Identifying the HSP sphere (of skin and/or the two or more solvents) may comprise using predicted/reference the HSP values.

### The skin

The term "skin" may refer to one, more, or all layers of the skin. Starting with the outermost layer going through to the innermost layer, the layers of the skin include: the epidermis, the dermis, and the hypodermis (subcutaneous fat layer). Preferably the skin refers to the epidermis. The epidermis can also be subdivided into layers. Starting with the outermost layers going through to the innermost layer, the layers of the epidermis are called the stratum corneum (SC), the stratum lucidum, the stratum granulosum, the stratum spinosum and the stratum basale. Thus, the term skin may refer to one or more layers selected from the stratum corneum, the stratum lucidum, the stratum granulosum, the stratum spinosum and the stratum basale.

### Matrikine

A matrikine is a bioactive signalling peptide that upregulates the production of proteins of the extracellular matrix (ECM), including at least fibrillin, fibronectin, decorin and collagen IV. Matrikines can be produced naturally within skin by the fragmentation of ECM proteins or synthesised.

Identifying the HSP sphere of the matrikine may comprise determining the HSP sphere of a composition comprising the matrikine, determining the HSP sphere of the phase of the composition comprising the matrikine (e.g., a part of the composition in which the matrikine is dissolved), determining the HSP sphere of an excipient composition comprising the matrikine, or determining the HSP sphere of the pure matrikine. A pure matrikine may be a matrikine of at least about 75% purity, at least about 80% purity, at least about 85% purity, at least about 90% purity, at least about 95% purity, at least about 96% purity, at least about 97% purity, at least about 98% purity, or at least about 99% purity.

The matrikine may be one matrikine, or a mixture of two or more matrikines.

The matrikine may be a lipo-matrikine (a lipophilic matrikine). A lipo-matrikine is a matrikine that has been modified so that is attached to a lipophilic group. The lipophilic group may be attached to the matrikine via a covalent bond.

The matrikine may have an amino acid sequence selected from the group consisting of: (i) Y-GPXG-Z (SEQ ID No. 7), (ii) Y-LSXX-Z (SEQ ID No. 8), (iii) Y-XXGD-Z (SEQ ID No. 9), (iv) Y-QTAV-Z (SEQ ID No. 10), (v) GHK (SEQ ID No. 5), and (vi) GQPR (SEQ ID No. 6),
wherein for (i) G denotes the amino acid glycine and P denotes the amino acid proline (as per the internationally recognised single letter code for amino acids), X denotes an amino acid selected from the group consisting of Lysine (K), Glutamic acid (E) and Serine (S), and mixtures thereof,
wherein for (ii) L denotes the amino acid Leucine and S denotes the amino acid Serine (as per the internationally recognised single letter code for amino acids), and X denotes an amino acid selected from the group consisting of Valine (V), Aspartic acid (D), Proline (P), Glycine (G), and mixtures thereof,
wherein for (iii) G denotes the amino acid Glycine and D denotes the amino acid Aspartic acid (as per the internationally recognised single letter code for amino acids), and X denotes an amino acid selected from the group consisting of Glutamic acid (E), Lysine (K), Leucine (L), Alanine (A), Isoleucine (I), Arginine (R) and mixtures thereof,
wherein for (iv) Q denotes the amino acid Glutamine, T denotes the amino acid Threonine, A denotes the amino acid Alanine, and V denotes the amino acid Valine,
wherein for (v), G denotes the amino acid Glycine, H denotes Histidine, and K denotes the amino acid Lysine (as per the internationally recognised single letter code for amino acids), and
wherein for (vi), G denotes the amino acid Glycine, Q denotes the amino acid Glutamine, P denotes the amino acid Proline, and R denotes the amino acid Arginine (as per the internationally recognised single letter code for amino acids), and
wherein at the N-terminal end, Y is selected from the group consisting of H, -CO-R¹, -SO₂-R¹ or a biotinyl group, and at the C-terminal end, Z is selected from the group consisting of OH, OR¹, NHR¹ or NR¹R².

In one embodiment, R¹ and R² are independently selected from the group consisting of alkyl, aryl, aralkyl, alkylaryl, alkoxy, saccharide and aryloxy group, which may be linear, branched, cyclical, polycyclic, unsaturated, hydroxylates, carbonylated, phosphorylated and/or sulphurous, said groups comprising from 1 to 24 carbon atoms and being capable of including one or more heteroatoms O, S and/or N.

In a preferred embodiment of the present invention the matrikine is modified at the N-terminal and/or the C-terminal end. Thus, modification may be a Y at the N-terminal end and/or a Z at the C-terminal end, wherein Y is selected from the group consisting of H, -CO-R¹, -SO₂-R¹ or a biotinyl group; and Z is selected from the group consisting of OH, OR¹, NHR¹ or NR¹R².

In a preferred embodiment of the present invention the tetrapeptide is any of the tetrapeptide referred to herein that has not been modified at the N-terminal and/or the C-terminal end. Thus, the peptide does not comprise a Y at the N-terminal end and/or a Z at the C-terminal end.

In a preferred embodiment the tetrapeptides of the present invention is selected from the group consisting of: Y-EKGD-Z (SEQ ID No. 13), Y-ELGD-Z (SEQ ID No. 14), Y-EAGD-Z (SEQ ID No. 15), Y-EIGD-Z (SEQ ID No. 16), Y-ERGD-Z (SEQ ID No. 17), Y-KEGD-Z (SEQ ID No. 18), Y-KLGD-Z (SEQ ID No. 19), Y-KAGD-Z (SEQ ID No. 20), Y-KIGD-Z (SEQ ID No. 21), Y-KRGD-Z (SEQ ID No. 22), Y-LEGD-Z (SEQ ID No. 23), Y-LKGD-Z (SEQ ID No. 24), Y-LAGD-Z (SEQ ID No. 25), Y-LIGD-Z (SEQ ID No. 26), Y-LRGD-Z (SEQ ID No. 27), Y-IEGD-Z (SEQ ID No. 28), Y-IKGD-Z (SEQ ID No. 29), Y-ILGD-Z (SEQ ID No. 30), Y-IAGD-Z (SEQ ID No. 31), Y-IRGD-Z (SEQ ID No. 32), Y-REGD-Z (SEQ ID No. 33), Y-RKGD-Z (SEQ ID No. 34), Y-RLGD-Z (SEQ ID No. 35), Y-RAGD-Z (SEQ ID No. 36), Y-RIGD-Z (SEQ ID No. 37), Y-AEGD-Z (SEQ ID No. 38), Y-AKGD-Z (SEQ ID No. 39), Y-ALGD-Z (SEQ ID No. 40), Y-AIGD-Z (SEQ ID No. 41) and Y-ARGD-Z (SEQ ID No. 42). In another embodiment, the tetrapeptides is selected from the group consisting of Y-EKGD-Z (SEQ ID No. 13), Y-LKGD-Z (SEQ ID No. 24), Y-IRGD-Z (SEQ ID No. 32) and Y-AKGD-Z (SEQ ID No. 39). In another embodiment the tetrapeptide is Y-EKGD-Z (SEQ ID No. 13). In another embodiment the tetrapeptide is Y-LKGD-Z (SEQ ID No. 24). In another embodiment the tetrapeptide is Y-IRGD-Z (SEQ ID No. 32). In another embodiment the tetrapeptide is Y-AKGD-Z (SEQ ID No. 39).

In a preferred embodiment the tetrapeptide combination of the present invention, tetrapeptide a) is selected from the group consisting of: Y-LSVD-Z (SEQ ID No. 43), Y-LSVP-Z (SEQ ID No. 44), Y-LSVG-Z (SEQ ID No. 45), Y-LSDV-Z (SEQ ID No. 46), Y-LSDP-Z (SEQ ID No. 47), Y-LSDG-Z (SEQ ID No. 48), Y-LSPV-Z (SEQ ID No. 49), Y-LSPD-Z (SEQ ID No. 50), Y-LSPG-Z (SEQ ID No. 51), Y-LSGV-Z (SEQ ID No. 52), Y-LSGD-Z (SEQ ID No. 53) and Y-LSGP-Z (SEQ ID No. 54). In another embodiment, the tetrapeptides is selected from the group consisting of Y-LSVD-Z (SEQ ID No. 43), Y-LSPG-Z (SEQ ID No. 51) and Y-LSPD-Z (SEQ ID No. 50). In another embodiment the tetrapeptide is Pal-LSVD-OH (SEQ ID No. 55). In another embodiment the tetrapeptide is Pal-LSPG-OH (SEQ ID No. 56). In another embodiment the tetrapeptide is Pal-LSPD-OH (SEQ ID No. 57).

In a further preferred embodiment of the present invention, the tetrapeptide is Y-GPKG-Z (SEQ ID No. 58). In a further preferred embodiment of the present invention the tetrapeptide is Y-GPEG-Z (SEQ ID No. 59). In a further preferred embodiment of the present invention the tetrapeptide is Y-GPSG-Z (SEQ ID No. 60).

The matrikine may be or comprise GHK (e.g., Pal-GHK (SEQ ID No. 75)) and/or GQPR (e.g., Pal-GQPR (SEQ ID No. 62)).

Where, at the N-terminal, Y is H then the amino acid is not modified. When, at the C-terminal, Z is OH then the amino acid is not modified. The tetrapeptide is thus not in derivatised form. When other than Y is H and Z is OH, then the tetrapeptide is derivatised. Derivation of the tetrapeptide is intended to increase the bioavailability of the peptide by improving the ability of the tetrapeptide to pass through the skin. An increase in bioavailability can also be achieved through encapsulation, microemulsions or through vectoring that increase the peptide delivery to the skin.

In a preferred embodiment of the present invention, R¹ and/or R² is an alkyl chain of from 1 to 24 carbon atoms, preferably a lipophilic alkyl chain of 3 to 24 carbon atoms.

In a further preferred embodiment of the present invention Y is an acyl group -CO-R¹ and Z is selected from the group consisting of OH, methoxy, ethoxy and NH₂. Z is preferably OH. In a further embodiment, Y is preferably independently selected from the group consisting of octanoyl (C8), decanoyl (C10), lauroyl (C12), myristoyl (C14), palmitoyl (C16), stearoyl (C18), biotinoyl, elaidoyl, oleoyle and lipoyle. In a preferred embodiment of the present invention, Y is selected from lauroyl (C12), myristoyl (C14) and palmitoyl (C16).

In a further preferred embodiment Z is OH and Y is independently selected from the group consisting of palmitoyl (C16), myristoyl (C14) and lauroyl (C12). Most preferably Y is palmitoyl (C16) and Z is OH.

The matrikine may comprise amino acids in the D- or L- configuration. The matrikine may comprise an acid C-terminus such as -CO2H. Alternatively, the matrikine may comprise an amide C-terminus such as -CONH2, -CONHR or CONR2, wherein R is an alkyl chain of preferably from 1 to 24 carbon atoms.

The amino acids making up the matrikine according to the invention may be optically pure, be made up of L or D isomers or a mixture of them. L isomers are those present in the natural state and may be preferred.

The present invention also envisages further derivatives of the matrikine, including for example modification and/or addition of a chemically functional group to one or more of the amino acids but without a change in the carbon skeletal. The present invention also envisages further analogues of the matrikine, including modification and/or addition of a chemically functional group to one or more of the amino acids with a change in the carbon skeletal and complexes of the matrikine with other species such as a metal ion (e.g., copper, zinc, manganese, magnesium, and others).

The matrikine may be in the form of a salt, including a hydrochloric salt, or an acetate.

### Two or more solvents for the matrikine

The skilled person will appreciate that the larger the number of different test solvents there are to choose from, the greater that chance of choosing a desirable solvent. Thus, the two or more test solvents may be three or more, four or more, five or more, six or more, seven or more, eight or more, nine or more, 10 or more, 11 or more, 12 or more 13 or more, 14 or more, 15 or more, 16 or more, 17 or more, 18 or more, 19 or more, 20 or more, 25 or more, 30 or more, 35 or more, 40 or more, 45 or more, or 50 or more test solvents.

A desirable solvent may be one that: (i) improves the delivery of the matrikine to the skin, (ii) improves the stability of the matrikine within a composition, or (iii) improves the delivery of the matrikine to the skin and improves the stability of the matrikine within a composition.

### Step (b) - Choosing at least one test solvent

The step of choosing at least one of the two or more test solvents may be dependent on the proximity of the chosen test solvent's HSP sphere to the HSP sphere of skin and/or the HSP sphere of the matrikine.

Preferably the HSP sphere of the chosen test solvent partially or completely overlaps with the HSP sphere of the matrikine, in order for the test solvent to dissolve the matrikine. Thus, the method according to the invention may comprise choosing the at least one test solvent because its HSP sphere partially or completely overlaps with the HSP sphere of the matrikine. The skilled person will appreciate that the closer the centre of the HSP sphere of the matrikine is to the centre of the HSP sphere of the test solvent, the more soluble the matrikine will be in a composition comprising the chosen solvent. The method according to the invention may comprise choosing the at least one test solvent because the centre of its HSP sphere is closer to the centre of the HSP sphere of the matrikine than the centre of the HSP spheres of non-chosen solvent(s). The method according to the invention may comprise choosing the at least one test solvent because it has a HSP sphere that overlaps with the HSP sphere of the matrikine. The method according to the invention may comprise choosing the at least one test solvent because it has a HSP sphere that overlaps with the HSP sphere of the matrikine and the centre of its HSP sphere is closer to the centre of the HSP sphere of the matrikine than the centre of the HSP sphere(s) of non-chosen solvent(s).

Preferably the HSP sphere of the chosen test solvent partially or completely overlaps with the HSP sphere of skin, in order for the solvent to encourage delivery of the matrikine to skin. Thus, the method according to the invention may comprise choosing the at least one test solvent because its HSP sphere partially or completely overlaps with the HSP sphere of skin. The skilled person will also appreciate that the closer the centre of the HSP sphere of skin is to the centre of the HSP sphere of the test solvent, the greater the amount of the matrikine that will be delivered to skin (if the chosen solvent is used to create a composition by dissolving the matrikine, and the matrikine is applied to skin). The method according to the invention may comprise choosing the at least one test solvent because the centre of its HSP sphere is closer to the centre of the HSP sphere of the skin than the HSP sphere(s) of non-chosen solvent(s). The method according to the invention may comprise choosing the at least one test solvent because it has a HSP sphere that overlaps with the HSP sphere of skin. The method according to the invention may comprise choosing the at least one test solvent because it has a HSP sphere that overlaps with the HSP sphere of skin and the centre of its HSP sphere is closer to the centre of the HSP sphere of the skin than the HSP sphere(s) of non-chosen solvent(s).

Two or more, three or more, or four or more (non)solvents with HSP values at opposite ends of the HSP sphere of skin or at opposite ends of the HSP sphere of the matrikine may be mixed together to form a solvent mixture that dissolves skin or the matrikine. Thus, in one embodiment, the step of choosing at least one test solvent may comprise choosing and mixing two or more (non) solvents, or three or more (non)solvents from a list of four or more, five or more, six or more, seven or more, eight or more, nine or more, 10 or more, 11 or more, 12 or more 13 or more, 14 or more, 15 or more, 16 or more, 17 or more, 18 or more, 19 or more, 20 or more, 25 or more, 30 or more, 35 or more, 40 or more, 45 or more, or 50 or more (non)solvents to form a solvent mixture, whereby the chosen (non)solvents have HSP values at opposite ends of the HSP sphere of skin or the HSP sphere of the matrikine.

In another embodiment, the step of choosing at least one of the two or more test solvents based on the proximity of its HSP sphere to the HSP sphere of skin, and/or the proximity of its HSP sphere to the HSP sphere of the matrikine may comprise using FFE.

### The composition

The composition according to the invention may comprise an excipient composition comprising the matrikine. The composition may further comprise a selection of one or more members of the group consisting of: emulsifiers, further peptides, matrix metalloproteinase inhibitors (MMPi), a skin conditioning agent, an antioxidant agent, a vitamin, a salicylic acid compound, a sunscreen, and other optional ingredients.

### Excipient composition

The matrikine of the present invention are provided by the supplier to the composition formulator as a concentrated excipient composition. The excipient composition is therefore a composition ingredient. The excipient composition comprises a matrikine at from 250 to 1650ppm. In an alternative embodiment, the excipient composition comprises from 0.1 to 50,000ppm. In a further alternative embodiment, the excipient composition comprises from 1 to 5,000ppm. In a further alternative embodiment, the excipient composition comprises from 10 to 500ppm.

The excipient composition, in addition to the matrikine, may optionally comprise one or more ingredients selected from the group consisting of: water, surfactants, diols, triols, glycerine, thickener and mixtures thereof. All suitable surfactants, diols (also known as glycols), triols, glycerine and thickener ingredients may be incorporated into the present excipient composition. Preferred surfactants for the excipient composition are selected from the group consisting of alkyl polyglucosides (prefereably decyl glucoside, coco glucoside, lauryl glucoside), sodium lauroyl lactylate, polysorbate 20, polysorbate 60, sorbitan laurate, PEG/PPG-18/18 dimethicone, Cetyl PEG/PPG-10/1 dimethicone, Polyglyceryl-4 isostearate, Hexyl laurate, steareth-21, steareth-2, and mixtures thereof.

Preferred diols are selected from the group consisting of; pentylene glycol, caprylyl glycol, butylene glycol, di-propylene glycol, ethylhexylglycerine, propanediol, hexenediol, glycerol, butylene glycol, propylene glycol, isoprene glycol, dipropylene glycol, pentylene glycol, hexylene glycol, polypropylene glycol, butylene glycol, polyethylene glycol, sorbitol, glucitol, mannitol, hydroxypropyl sorbitol, erythritol, threitol, pentaerythritol, xylitol, and mixtures thereof.

Preferred triols are selected from the group consisting of: hexanetriol, glycerine, ethoxylated glycerin, propoxylated glycerin, and mixtures thereof.

Preferred thickeners are selected from the group consisting of: xanthan gum, ammonium acryloyldimethyltaurate/vinyl pyrrolidone copolymer, dimethicone crosspolymer, carbomer, hydroxyethyl cellulose, polyacrylamide, sodium polyacrylate, polyacrylate crosspolymer-6, acrylates/beheneth-25 methacrylate copolymer, Acrylates/C10-30 alkyl acrylate crosspolymer, and mixtures thereof.

### Cosmetic Composition

The composition of the invention is preferably a cosmetic composition, such as a skin care composition, e.g., a facial skincare cosmetic composition. Thus, the present invention also encompasses cosmetic compositions comprising one or more solvents of the present invention (e.g., a silicone, an alkoxylated sugar ether and/or an isosorbide) and a matrikine.

The cosmetic composition of the present invention may be aqueous or non-aqueous and comprise a single-phase system or multiple phase system. The composition may include but is not limited to liquids, gels, balms, oils or solids. Single or multiple phase compositions are envisaged. Multiple phase systems include but are not limited to microemulsions, emulsions, and products with discrete separate phases. Emulsions include water-in-oil, oil-in-water emulsions and multiple emulsions (water in oil in water or oil in water in oil for example). Products with discrete separate phases include bi or triphasic systems where the individual water or oil phases can be visibly seen. Preferably the composition is a gel or an emulsion. Preferably the emulsion is an oil-in-water emulsion.

Where the composition is aqueous, it preferably comprises from 10% to 99.9% by weight water. In a preferred embodiment, aqueous compositions comprise from 20% to 80 % by weight water. In a preferred embodiment, aqueous compositions comprise from 40% to 70% by weight water. Where the composition is non-aqueous it preferably comprises 0% to up to 10% water, more particularly from 0.1 to 8%, most preferably from 0.5 to 5% water.

Where the composition is an emulsion it comprises an oil and a water phase. The oil phase of an emulsion can be provided by any suitable oily component. Suitable oils for the oil phase may comprise for example: a) hydrocarbon oils, paraffin or mineral oils; b) waxes, such as beeswax or paraffin wax; c) natural oils, such as sunflower oil, apricot kernel oil, shea butter or jojoba oil; d) silicone oils, such as dimethicone, silicone elastomer, cyclomethicone or cetyl dimethicone; e) fatty acid esters and ethers, such as isopropyl palmitate or isopropyl myristate and polypropylene glycol-15 stearyl ether; f) fatty alcohols, such as cetyl alcohol or stearyl alcohol; or g) mixtures thereof, for example, the blend of waxes available commercially under the trade name Cutina (BASF).

The emulsion may comprise 0.1% to 55% by weight of the emulsion of oil phase. In one embodiment, the emulsion may comprise 3% to 25% by weight of the emulsion of oil phase, more preferably from 5% to 20% by weight of the emulsion of oil phase. In an alternative embodiment, the emulsion may comprise 10% to 50% by weight of the emulsion of oil phase, more preferably from 25-50% by weight of the emulsion of oil phase.

Preferably the oil phase of the emulsion comprises oil at a level between 50% and 100% by weight of the oil phase. More preferably the oil phase comprises oil at a level of from 60% to 100%, more preferably from 70% to 100%, and even more preferably from 80% to 100% by weight of the oil phase. Alternatively, the oil phase of the emulsion may comprise a combination of oil, wax or butter. Waxes and butters are hydrocarbons that consist of long aliphatic alkyl chains and may include aromatic groups. They are generally lipophilic and typically solid or malleable at room temperature. Melting points vary depending on the alkyl chain, chain length and associations. Silicone waxes are preferred type of suitable wax based on alkylmethylsiloxane. Oils are typically lipophilic and liquid at room temperature with lower molecular weights than waxes. Where present wax or butter may be present at up to 40% of the oil phase of the emulsion. More preferably the oil phase may contain wax or butter at levels of up to 20% of the oil phase of the emulsion. In an alternative embodiment the oil phase may contain wax or butter at levels of up to 10% of the oil phase of the emulsion.

Preferably the oil phase of the water-in-oil emulsion comprises a silicone oil. Where present, the silicone-containing oil phase preferably comprises an organo polysiloxane oil. The organopolysiloxane oil for use in the composition may be volatile, non-volatile, or a mixture of volatile and non-volatile silicones. The term "nonvolatile" as used in this context refers to those silicones that are liquid or gel under ambient conditions and have a flash point (under one atmosphere of pressure) of greater than 100°C. The term "volatile" as used in this context refers to all other silicone oils. Suitable organopolysiloxanes can be selected from a wide variety of silicones spanning a broad range of volatilities and viscosities. Examples of suitable organopolysiloxane oils include polyalkylsiloxanes, cyclic polyalkylsiloxanes, and polyalkylarylsiloxanes.

Preferred for use herein are organopolysiloxanes selected from the group consisting of: polyalkylsiloxanes, alkyl substituted dimethicones, cyclomethicones, trimethylsiloxysilicates. dimethiconols, polyalkylaryl siloxanes, and mixtures thereof. More preferred for use herein are polyalkylsiloxanes and cyclomethicones. Preferred among the polyalkylsiloxanes are dimethicones.

Alternatively, the silicone oil may be a silicone elastomer. Suitable for use herein are silicone elastomers which can be emulsifying or non-emulsifying crosslinked siloxane elastomers or mixtures thereof. No specific restriction exists as to the type of curable organopolysiloxane composition that can serve as starting material for the crosslinked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon- bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane and condensation-curing organopolysiloxane compositions which cure in the presence of an organotin compound or a titanate ester.

Preferably the oil phase comprises silicone, and most preferably, a silicone elastomer. Preferably, the emulsion composition includes from 20% to 35%, by weight of the emulsion composition, of the silicone elastomer raw material.

When the composition is a water-in-oil emulsion it preferably comprises an emulsifier. In a preferred embodiment, the composition comprises from 0.1% to 10% emulsifier, more preferably from 0.25% to 7.5%, still more preferably from 0.5% to 5%, emulsifier by weight of the composition. The emulsifier helps disperse and suspend the aqueous water phase within the oil phase.

### Emulsifiers

The composition of the present invention may comprise an emulsifier. Suitable emulsifiers include all those suitable for the purpose and known by those skilled in the art for use in skin care products. Preferably these emulsifiers have an HLB value of or less than 14, more preferably from 2 to 14, and still more preferably from 4 to 14.

Silicone emulsifiers are preferred. A wide variety of silicone emulsifiers are useful herein. These silicone emulsifiers are typically organically modified organopolysiloxanes, also known to those skilled in the art as silicone surfactants. The skilled person will appreciate that organically modified organopolysiloxanes comprise one or more hydrophilic groups as well as silicone. Useful silicone emulsifiers include dimethicone copolyols. These materials are polydimethyl siloxanes which have been modified to include polyether side chains such as polyethylene oxide chains, polypropylene oxide chains, mixtures of these chains, and chains comprising moieties derived from both ethylene oxide and propylene oxide. Other examples include alkyl-modified dimethicone copolyols, i.e., compounds which comprise C2-C30 pendant side chains. Still other useful dimethicone copolyols include materials having various cationic, anionic, amphoteric and zwitterionic pendant moieties.

Non-limiting examples of dimethicone copolyols and other silicone surfactants useful as emulsifiers herein include polydimethylsiloxane polyether copolymers with pendant polyethylene oxide side chains, polydimethylsiloxane polyether copolymers with pendant polypropylene oxide side chains, polydimethylsiloxane polyether copolymers with pendant mixed polyethylene oxide and polypropylene oxide side chains, polydimethylsiloxane polyether copolymers with pendant mixed poly (ethylene) (propylene) oxide side chains, polydimethylsiloxane polyether copolymers with pendant organobetaine side chains, polydimethylsiloxane polyether copolymers with pendant carboxylate side chains, polydimethylsiloxane polyether copolymers with pendant quaternary ammonium side chains; and also further modifications of the preceding copolymers comprising pendant C2-C30 straight, branched, or cyclic alkyl moieties. A particularly preferred emulsifier is PEG/PPG-18/18 dimethicone.

Suitable, cetyl dimethicone copolyol is commercially available as a mixture with polyglyceryl-4 isostearate (and) hexyl laurate or as a mixture with hexyl laurate and polyglyceryl-3 oleate. Other nonlimiting examples of dimethicone copolyols also include lauryl dimethicone copolyol, dimethicone copolyol acetate, diemethicone copolyol adipate, dimethicone copolyolamine, dimethicone copolyol behenate, dimethicone copolyol butyl ether, dimethicone copolyol hydroxy stearate, dimethicone copolyol isostearate, dimethicone copolyol laurate, dimethicone copolyol methyl ether, dimethicone copolyol phosphate, and dimethicone copolyol stearate.

Among the non-silicone-comprising emulsifiers useful herein are various non-ionic and anionic emulsifying agents such as sugar esters and polyesters, alkoxylated sugar esters and polyesters, Cl-C30 fatty acid esters of C1-C30 fatty alcohols, alkoxylated derivatives of C1-C30 fatty acid esters of C1-C30 fatty alcohols, alkoxylated ethers of C1-C30 fatty alcohols, polyglyceryl esters of C1-C30 fatty acids, C1-C30 esters of polyols, C1-C30 ethers of polyols, alkyl phosphates, polyoxyalkylene fatty ether phosphates, fatty acid amides, acyl lactylates, soaps, and mixtures thereof. Nonlimiting preferred examples of these non-silicon-comprising emulsifiers include: polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, PEG-100 stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, steareth-20, ceteareth-20, PPG-2 methyl glucose ether distearate, ceteth-10, diethanolamine cetyl phosphate, glyceryl stearate, PEG-100 stearate, and mixtures thereof.

### Further peptides

The compositions of the present invention may comprise further peptides. Preferably said additional peptides are selected from the group consisting of dipeptides, tripeptides, additional tetrapeptides, pentapeptides, and mixtures thereof. By tripeptides, it is meant compound comprising an uninterrupted sequence of three amino acids. By tetrapeptides, it is meant a compound comprising an uninterrupted sequence of four amino acids and when using further tetrapeptides, the tetrapeptides are referred to as 'additional tetrapeptides'. By pentapeptide it is meant a compound comprising an uninterrupted sequence of five amino acids.

Dipeptides:
The compositions of the present invention may comprise a dipeptide selected from the group consisting of acetyl dipeptide 1 cetyl ester, acetyl dipeptide 3 aminohexanoate, azelaoyl bisdipeptide 10, coumaroyl dipeptide 3, dicetyl dipeptide 9, dipeptide diamino butyroyl benzylamide diacetate, dipeptide 1, dipeptide 10, dipeptide 11, dipeptide 12, dipeptide 15, dipeptide 16, dipeptide 17,
dipeptide 18, dipeptide 19, dipeptide 2, dipeptide 20, dipeptide 3, dipeptide 4, dipeptide 5, dipeptide 6,
dipeptide 7, dipeptide 8, dipeptide 8 HCL, dipeptide 9, hexanoyl dipeptide 3 norleucine acetate,
methyl undecylenoyl dipeptide 16, nicotinoyl dipeptide 22, nicotinoyl dipeptide 23, nicotinoyl dipeptide 24, nicotinoyl dipeptide 26, oleoyl dipeptide 15, palmitoyl dipeptide 10, palmitoyl dipeptide 13, palmitoyl dipeptide 17, palmitoyl dipeptide 5 diaminobutyroyl hydroxythreonine, palmitoyl dipeptide 5 diaminohydroxybutyrate, palmitoyl dipeptide 7, and mixtures thereof.

Dipeptides are preferably incorporated into the cosmetic composition of the present invention at a level of from 0.1 to 50000ppm, more preferably from 1 to 5000 ppm, most preferably from 10 to 500ppm.

Tripeptides:
The emulsions of the present invention preferably comprise a tripeptide. Said tripeptide may be naturally occurring or of synthetic origin. Suitable tripeptides include tripeptide 1, 2, 3, 4, 5, 6, 7, 8, 9, 10,11, 12, 13, 14, 15, 16, 17, 18, 19, 20, 21, 22, 23, 24, 25, 26, 27, 28 ,29, 30, 31, 32, 33, 34, 35, 36, 37, 38, 39, 40, 41, 42, 43, 44, 45, 46, derivatives thereof and mixtures thereof.

Particularly preferred tripeptides comprise one or more His-based tripeptides. However, another suitable tripeptide may be Arg-Lys-Arg. Particularly preferred tripeptides are based on the structure Gly-His-Lys and its analogs and derivatives thereof. These are collectively known herein as GHK-tripeptides. Indeed, the preferred tripeptide in accordance with this aspect of the invention has this exact sequence of amino acids. Analogs of the preferred tripeptide useful herein include those in which one or more of the three amino acids are reorganized or rearranged within the sequence (e.g., Gly-Lys-His) and/or where no more than two amino acids are substituted (e.g., His-Ala-Orn). However, most preferably, amino acids substituted for Gly include an aliphatic side chain such as, without limitation, beta-Ala, Ala, Val, Leu, Pro, Sarcosine (Sar) and Ile. Most preferred are Ala, Leu and Ile. The most preferable amino acid substituted for Lys or His include those having a side chain that includes, predominantly, a charged nitrogen at a pH of 6, such as, without limitation, Pro, Lys, Arg, His, Desmosine and Isodesmosine. Most preferably, Lys is replaced with Orn, Arg, or Citrulline.

Derivatives are also considered to be encompassed by the term GHK-tripeptides in accordance with the present invention, (and therefore also the more generic term tripeptides). Derivatives of GHK-tripeptides in accordance with the present invention include derivatives of the substituted and rearranged tripeptides described herein. These derivatives include, inter alia, acyl-derivatives, which are tripeptides substituted with one or more straight-chain or branched-chain, long or short chain, saturated or unsaturated, substituted with a hydroxy, amino, acyl amino, sulfate or sulfide group, or unsubstituted, which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO₃H, SH or S-S.

His-based tripeptides include at least one Histadine amine acid. The other two amino acids in the sequence may be the same or different. Thus, contemplated are, without limitation, His-Xaa-Xaa, His-Xaa-Xbb, His-Xbb-Xaa, Xbb-His-Xbb, Xbb-His-Xaa, Xaa-His-Xbb, Xaa-Xaa-His, Xaa-Xbb-His, Xbb-Xaa-His and Xbb-Xbb-His, where Xaa and Xbb are two different amino acids, although either can be His. Preferably, at least one of the other amino acids is Gly, beta-Ala, Ala, Val, Leu, Pro, Sarcosine (Sar) or Ile. Preferably, at least one of the other amino acids is Pro, Lys, Arg, His, Desmosine and Isodesmosine. Most preferably, Lys is replaced with Orn, Arg, or Citrulline.

Derivatives are also considered to be encompassed by the term His-based tripeptides in accordance with the present invention, (and therefore also the more generic term tripeptides). These derivatives include, inter alia, acyl-derivatives, which are tripeptides substituted with one or more straight-chain or branched-chain, long or short chain, saturated or unsaturated substituted or unsubstituted acyl group(s) having from 1 to 29 carbon atoms. The acyl groups which can be used are the same as those described for the GHK-tripeptides.

Particularly preferred embodiments of tripeptides in accordance with the present invention include N-Acyl-Gly-His-Lys and most preferably, N-Palmitoyl-Gly-His-Lys. Preferred commercially available tripeptide and tripeptide derivative comprising compositions include Biopeptide-CL from SEDERMA, Maxilip(R) from SEDERMA, Biobustyl(R) from SEDERMA.

The tripeptides (or an excipient composition comprising the tripeptide), where included, are preferably incorporated into the cosmetic composition in amounts of from 0.10ppm to 10,000ppm, preferably from 0.50ppm to 5,000ppm, more preferably from 1ppm to 1000ppm, and most preferably from 1ppm to 500ppm. These are again based on a % w/w basis. Thus 100,000ppm is 10% by weight of the emulsion.

Tetrapeptides:
The cosmetic composition may comprise an additional tetrapeptide. These may be one or more rigin-based tetrapeptides, one or more ALAMCAT-tetrapeptides or mixtures thereof. These tetrapeptides may be naturally occurring or of synthetic origin. Suitable tetrapeptides for use in the present composition include those selected from the group consisting of well-known tetrapeptide 1, 2, 3, 4, 5, 6, 7, 8, 9, 10, 11, 12,13, 14, 15, 16, 17, 18, 19 ,20, 21, 22, 23, 24, 25, 26, 27, 28, 29, 30, 34, 35, derivatives thereof, and mixtures thereof.

Rigin-based tetrapeptides in accordance with the present invention are based on the structure Gly-Gln-Pro-Arg (Rigin) and include its analogs and derivatives thereof. Rigin is an additional tetrapeptide. Analogs of the tetrapeptide rigin useful in accordance with the present invention include those in which one or more of the four amino acids are reorganized or rearranged within the sequence and/or where no more than two of the amino acids are substituted (e.g., Ala-Gln-Thr-Arg. More preferably, at least one of the amino acids within the sequence is Pro or Arg and most preferably the tetrapeptide includes both Pro and Arg although their order and position may vary. The amino acid substitutions can be from amongst any amino acid as defined herein. Particularly preferred rigin-based tetrapeptides include Xaa-Xbb-Arg-Xcc (SEQ ID No. 63), Xaa-Xbb-Xcc-Pro (SEQ ID No. 64), Xaa-Xbb-Pro-Arg (SEQ ID No. 65), wherein Xaa-Xbb-Pro-Xcc (SEQ ID No. 66), Xaa-Xbb-Xcc-Arg (SEQ ID No. 67), Xaa, Xbb and Xcc may be the same or different and selected from the following Xaa is Gly or the amino acids that may be substituted therefore, Xbb is Gln or the amino acids that may be substituted therefore and Xcc may be Pro or Arg or the amino acids substituted therefore. The most preferable amino acids substituted for Gly include an aliphatic side chain such as, without limitation, beta-Ala, Ala, Val, Leu, Pro, Sarcosine (Sar) and Ile. The most preferable amino acids substituted for Gln include a side chain that includes an amine group that is predominantly uncharged at neutral pH (pH 6-7) such as, without limitation, Asn, Lys, Orn, 5-hydroxyproline, Citrulline and Canavanine. When Arg is substituted, it is preferably replaced with an amino acid having a side chain that includes, predominantly, a charged nitrogen at a pH of 6, such as, without limitation, Pro, Lys, His, Desmosine and Isodesmosine.

Derivatives are also considered to be encompassed by the term rigin-base tetrapeptides, (and therefore also the more generic term tetrapeptides). Derivatives include derivatives of the substituted and rearranged rigin-based tetrapeptides described herein. These derivatives include, inter alia, acyl-derivatives, which are tetrapeptides substituted with one or more straight-chain or branched-chain, long or short chain, saturated or unsaturated, substituted with a hydroxy, amino, amino acyl, sulfate or sulfide group or unsubstituted having from 1 to 29 carbon atoms. N-acyl-derivatives include those acyl groups which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or sulphur comprising groups such as, without limitation SO3H, SH or S-S.

Derivatives are also considered to include peptide-divalent ion complexes. Cu2+-peptide derivatives are preferred. Cu2+-peptide derivatives may provide an increased biological effect compared to the peptide alone.

ALAMCAT tetrapeptides are tetrapeptides which include at least one amino acid including an aliphatic group comprising side chain. These amino acids include, without limitation, Gly, beta-Ala, Ala, Val, Leu, Sarcosine (Sar) and Ile. These tetrapeptides also include at least one amino acid including at least one NH2 comprising side chain. These amino acids include a side chain that has an amine group that is predominantly uncharged at neutral pH (pH 6-7) such as, without limitation, Gln, Asn, Lys, Orn, 5-hydroxyproline, Citrulline and Canavanine. The ALAMCAT-tetrapeptides also include at least one amino acid having at least one side chain including at least one cationic amine (predominant species is charged such as NH3+, NH2+, etc.-basic amino acids which are positively charged at pH 6.0). These amino acids include, without limitation, Pro, Arg, Lys, His, Desmosine and Isodesmosine. The remaining amino acid can be any amino acid, but is preferably one comprising an alphatic group, pendant amino group or pendant cationic group. Derivatives are also considered to be encompassed by the term ALAMCAT-tetrapeptides in accordance with the present invention, (and therefore also the more generic term tetrapeptides). These derivatives include, inter alia, acyl-derivatives, which are tetrapeptides substituted with one or more straight-chain or branched-chain, substituted or unsubstituted long or short chain, saturated or unsaturated acyl group(s) having from 1 to 29 carbon atoms. The acyl groups which can be used are the same as those described for the rigin-based tetrapeptides.

Preferred embodiments include Peptide E, arg-ser-arg-lys (SEQ ID No. 68), N-acyl-Gly-Gln-Pro-Arg peptides (SEQ ID No. 69), most preferably N-palmitoyl-Gly-Gln-Pro-Arg (SEQ ID No. 69).

Preferred commercially available sources of tetrapeptides include RIGIN, EYELISS, Haloxyl, and MATRIXYL 3000, which comprise between 50 to 500 ppm of palmitoyl-Gly-Gln-Pro-Arg (SEQ ID No. 69), and other ingredients, such as peptides, chalcones and an excipient, commercially available from SEDERMA, France. Tego Pep 417 available from Evonik. These may be used to produce compositions of the present invention by adding thereto at least one tripeptide as described herein.

The additional tetrapeptides when used are preferably incorporated into the cosmetic composition in amounts from 0.1 ppm (0.00001% w/w also referred to herein as "weight percent", "weight %" or simply by weight) to 10,000 ppm (0.5% w/w), preferably from 0.5 ppm to 1000 ppm (0.05% w/w), and most preferably from 1 ppm to 500ppm by weight of the composition.

The combination of tripeptides and additional tetrapeptides, can be particularly preferred. When present, the preferred ratio of additional tetrapeptide to tripeptide, or indeed the ratio of molecules having four amino acids to those having three amino acids can range from 100:1 to 1:100; more preferably from 50:1 to 1:50, even more preferably from 30:1 to 1:30 and even more preferably between 10:1 to 1:10. Most preferably, the ratio of additional tetrapeptide to tripeptide ranges from between 3:1 to 1:3. These ratios are on a weight basis (% w/w-e.g. mg of pure peptide per Kilogram in the final formulation). In a particularly preferred embodiment, the amount of tripeptide used is greater than the amount of additional tetrapeptide used when considered in terms of their amounts in parts per million, again based on overall weight of the composition. In a particularly preferred embodiment, the cosmetic composition of the present invention comprises an additional tetrapeptide of the sequence Gly-Gln-Pro-Arg, its analogs and derivatives in combination with one or more tripeptide of the sequences Gly-His-Lys, its analogs and derivatives.

### Pentapeptides

The compositions of the present invention may optionally comprise a pentapeptide, derivatives of pentapeptides, and mixtures thereof. As used herein, "pentapeptides" refers to both the naturally occurring pentapeptides and synthesized pentapeptides. Also useful herein are naturally occurring and commercially available compositions that comprise pentapeptides. Suitable pentapeptides are those selected from the group consisting of pentapeptide 1, 4, 5, 6, 7, 8, 9, 10, 11, 12, 13, 14, 15, 16, 17, 18, 19, 21, 22, 23, 24, 25, 26, 28, 29, 30, 31, 33, 34, 35, 36, 38, 39, derivatives thereof, and mixtures thereof.

Suitable pentapeptides for use herein are the pentapeptide, lys-thr-thr-lys-ser (SEQ ID No. 70), Arg-asp-lys-tyr-val (pentapeptide-1 (SEQ ID No. 71)) and derivatives thereof. A preferred commercially available pentapeptide derivative-comprising composition is Matrixyl which comprises 100 ppm of palmitoyl-lys-thr-thr-lys-ser (SEQ ID No. 72) and is commercially available from Sederma, France.

The pentapeptides and/or pentapeptide derivatives where present in the cosmetic composition are preferably included at amounts of from 0.1 ppm (0.00001% w/w t) to 10,000 ppm (0.5% w/w), preferably from 0.5 ppm to 1000 ppm (0.05% w/w), and most preferably from 1 ppm to 500ppm (0.025% w/w) by weight of the composition.

### Matrix metalloproteinase inhibitors (MMPi)

The term "matrix metalloproteinase inhibitor" relates to all molecules and/or plant or bacterial extracts having an inhibitory activity on at least one of the matrix metalloproteinases expressed or synthetized by or in the skin. The family of the matrix metalloproteinases is formed of several well-defined groups on the basis of their resemblance regarding structure and substrate specificity (Woessner J. F. 1991, Faseb Journal, vol. 5,-, 2145). Among these groups, there are collagenases able to degrade fibrillar collagens (MMP-1 or interstitial collagenase, MMP-8 or neutrophil collagenase, MMP- 13 or collagenase 3, MMP-18 or collagenase 4), gelatinases degrading type IV collagen or other denatured collagen form (MMP-2 or A gelatinase (72 kDa), MMP-9 or B gelatinase (92 kDa)), stromelysins (MMP-3 or stromelysin 1, MMP- 10 or stromelysin 2, MMP-11 or stromelysin 3) whose broad spectrum of activity targets proteins of the extracellular matrix such as glycoproteins (fibronectin, laminin), proteoglycanes etc., matrilysin (MMP-7), metalloelastase (MMP- 12) or metalloproteinases (MMP- 14, MMP- 15, MMP- 16 and MMP- 17). Metalloproteinases (MMPs) are proteases that use a metal, (mostly zinc) coordinated to 3 cysteine residues and to a methionine in their active site, that degrade macromolecular components of the extracellular matrix and of basal layers at neutral pH (collagen, elastin, etc ...). This group of enzymes is inactivated by metal chelators. The principal activity regulators of MMPs are the tissue inhibitors of metalloproteinases or TIMPs such TIMP-I, TIMP-2, TIMP-3 and TIMP-4 (Woessner J. F., Faseb Journal, 1991). Furthermore, MMP expression is also regulated by growth factors, cytokines, oncogene products (ras, jun), or also matrix constituents.

The term "matrix metalloproteinase inhibitors" according to the present invention means all molecules able to reduce the MMP's activity regarding the gene expression (transcription and translation) or regarding the activation of the zymogen form of the MMP, or else regarding the local control of active forms. Furthermore, the metalloproteinase inhibitors according to the present invention can also be MMP-1 inhibitors of natural or synthetic origin. The terms "natural origin" or "synthetic origin" mean both a metalloproteinase inhibitor at a pure state or in solution at different concentrations, but natural origin termed inhibitors are obtained by different extraction methods from a natural element (for example lycopene from a tomato) whereas the inhibitors of synthetic origin are all obtained via chemical synthesis

Preferred MMPi are selected from the group consisting of: retinoid, N-acetyl cysteine, glutathione, 2-furildioxime, vitamin C, flavones, isoflavones, hydrolysed rice protein, alfalfa extract, white lupin, zizyphus jujube extract, dihydroxy methyl chromone, kudzu extract, vitis vinifera extract, Oenothera biennis extract Anogeissus leiocarpus extract, and mixtures thereof.

Where present, MMPi are present at a level of from 0.01% to 10%, more preferably 0.1% to 5% and most preferably from 0.5% to 2.5% by weight of the cosmetic composition.

### Skin Conditioning Agent

The compositions of the present invention may optionally comprise a skin conditioning agent. Said skin conditioning agents may preferably be selected from the group consisting of: humectants, emollients, moisturisers, and mixtures thereof. Where present, they are preferably present at a level of from 0.01% to 20%, more preferably from 0.1% to 10%, most preferably from 0.5% to 7% by weight of the cosmetic composition.

Preferred skin conditioning agents are selected from the group consisting of: guanidine, urea, glycolic acid and glycolate salts, salicylic acid, lactic acid and lactate salts, aloe vera, shea butter, polyhydroxy alcohols, such as sorbitol, mannitol, xylitol, erythritol, glycerol, hexanetriol, butanitriol, (di) propylene glycol, butylene glycol, hexylene glycol, polyethylene glycol, sugars (e.g. fructose, glucose, xylose, honey, mannose, xylose), gluconodeltalactone, and starches and their derivatives, pyrrolidone, carboxylic acid, hyaluronic acid and salts thereof, lactamide monoethanolamine, acetamide monoethanolamine, panthenol, allantoin, and mixtures thereof.

More preferably said skin conditioning agent is selected from the group consisting of: glycerine, arabinogalactan, butylene glycol, hyaluronic acid, shea butter, propylene glycol, ethylhexyl glycerine, hyaluronate and mixtures thereof.

### Antioxidant Agent

The compositions of the present invention may optionally comprise an antioxidant agent. Suitable antioxidant agents may include: a) ascorbic acid its salts, esters, glucosides and glucosamines, particularly sodium ascorbyl phosphate, magnesium ascorbyl phosphate and ascorbyl palmitate b) vitamin E (tocopherol) and its esters, particularly tocopheryl acetate, as well as Dimethyl methoxy chromanol which is a synthetic analogue of gamma tocopherol, available from Lipotec S.A. polygon Industrial Camri Ral, under the tradename Lipochroman-6 c) herbal extracts, particularly gingko biloba, such as that available under the trade name "Gingko Biloba Leaf Powder" from Univar PLC, morus alba, such as that available under the trade name "Mulberry Concentrate" from Solabia, origanum vulgare, such as that available under the trade name "Pronalen Origanum HSC" from S Black Ltd, panax ginseng, such as that available under the trade name "Panax ginseng 1.1 extract 4294"from S Black Ltd or "Phytexcell Panax ginseng" available from Croda Chemicals Ltd, birch extract such as those available from Cosmetochem (U. K.) Ltd under the trade names "Super Herbasol Extract Birch" and "HP Herbasol Betula" and those available from Blagden Chemicals under the tradenames "Phytelene of Birch" and "Aqueous Spray Dried Birch", camellia sinensis, such as that available under the trade name "Herbal Extract Green Tea 75% Solids" from Nichimen Europe, rosmarinus officinalis, such as that available under the trade name "Pronalen Rosemary" from S. Black, Acerola cherry powder, such as that available as Acerola PE from Gee Lawson, Emblica extract sold under the tradename Emblica ^{™} by Merck Speciality chemicals, and Grape Seed oil, such as that available from Chesham Chemicals Limited.

The amounts of antioxidant agents used in the cosmetic composition are expressed as dry weights, as understood by a man skilled in the art. The total amount of antioxidant agents optionally present in the composition may range from 0.005% to 10% by weight, preferably 0.5% to 5%, most preferably 0.2% to 1.5% by weight of the composition.

Particularly preferred synergistic combinations of antioxidant agents suitable for inclusion in the cosmetic composition of the present invention are two or more members selected from the group consisting of: i) panax ginseng, morus alba and magnesium ascorbyl phosphate; ii) panax ginseng, morus alba and sodium ascorbyl phosphate; iii) panax ginseng, morus alba and rosmarinus officinalis; iv) ginkgo biloba, phyllanthus emblica and Dimethylmethoxy chromanol; v) morus alba, camellia sinensis and dimethylmethoxy chromanol; vi) morus alba, camellia sinensis and tocopheryl acetate; vii) panax ginseng, morus alba and origanum vulgare.

In these preferred combinations (a) the panax ginseng is preferably present in an amount of 0.005% to 0.1%, more preferably 0.01% to 0.05% by weight of the composition; (b) the morus alba is preferably present in an amount of 0.0005% to 0.01%, more preferably 0.001% to 0.005% by weight of the composition; (c) the sodium,magnesium ascorbyl phosphate or ethyl ascorbic acid is preferably present in an amount of 0.05% to 2.5%, preferably 0.1% to 2%, most preferably 0.15% to 1.5% by weight of the composition;(d) the rosmarinus officinalis or origanum vulgare or phyllanthus emblicais preferably present in an amount of 0.01% to 0.5%, more preferably 0.05% to 0.2% by weight of the composition e) the dimethylmethoxy chromanol is preferably present in an amount of 0.0005% to 0.1%, more preferably from 0.005% to 0.05% by weight of the composition ; f) the camellia sinensis is preferably present in an amount of 0.005% to 0.2%, more preferably from 0.01% to 0.1% and the g) Tocoperol acetate is preferably present in an amount of 0.01 to 0.5%, more preferably from 0.05% to 0.25%

### Vitamins

The compositions of the present invention may comprise one or more vitamins. The compositions may comprise ascorbates, for example vitamin C, vitamin C derivatives, ascorbic acid, ascorbyl glucoside, ascorbyl palmitate, magnesium ascorbyl phosphate, sodium ascorbyl phosphate and ethyl ascorbic acid. The composition may comprise vitamin B, vitamin B derivatives, vitamin B1 to vitamin B12 and their derivatives. In a further embodiment the composition comprising the Vitamin B3 derivative niacinamide.

In an alternative embodiment of the present the cosmetic composition comprises vitamin K, vitamin K derivatives, vitamin H, vitamin D, vitamin D derivatives and mixtures thereof. In an alternative embodiment of the present the cosmetic composition comprises vitamin E, vitamin E derivatives such as tocopherol and tocopheryl acetate, and provitamins thereof, such as panthenol and mixtures thereof.

In a further embodiment the present cosmetic composition comprises retinoid compounds, including retinoic acid, retinaldehyde, retinol and derivatives thereof. In one embodiment the cosmetic composition comprises retinyl palmitate, retinyl acetate, retinyl retinoate, retinyl proprionate, retinyl ascorbate, retinyl linoleate, retinyl retinoate, retinyl sunflowerseedate and mixtures thereof.

The vitamin compounds may be included as the substantially pure material, or as an extract obtained by suitable physical and/or chemical isolation from natural (e. g. plant) sources. In one embodiment, when vitamin compounds are present in the compositions of the instant invention, the emulsion compositions comprise from about 0.0001% to 50%, more preferably from 0.001% to 10%, still more preferably from 0.01% to 8%, and still more preferably from 0.1% to 5%, by weight of the composition, of the vitamin compound.

### Salicylic Acid Compound

The compositions of the present invention may comprise a salicylic acid compound, its esters, its salts, or combinations thereof. In one embodiment of the compositions of the present invention, the salicylic acid compound preferably comprises from 0.0001% to 25%, more preferably from 0.001% to 15%, even more preferably from 0.01% to 10%, still more preferably from 0.1% to 5%, and even more preferably from 0.01% to 2%, more preferably 0.1% to 2% by weight of the composition, of salicylic acid.

### Sunscreen

The compositions of the present invention may optionally comprise a sunscreen component. The sunscreen may comprise organic or inorganic sun filters or a combination of the two. Suitable inorganic sun filters include those selected from the group consisting of: microfine titanium dioxide, microfine zinc oxide, boron nitride and, mixtures thereof.

Suitable organic sunscreens include those selected from the group consisting of: a) p-aminobenzoic acids, their esters and derivatives (for example, 2ethylhexyl p-dimethylaminobenzoate), b) methoxycinnamate esters (for example, 2-ethylhexyl p-methoxycinnamate, 2-ethoxyethyl p-methoxycinnamate or a, p-di- (p-methoxycinnamoyl)-a'- (2ethylhexanoyl)-glycerin, c) benzophenones (for example oxybenzone), d) dibenzoylmethanes such as 4- (tert-butyl)-4'-methoxydibenzoylmethane, e) 2-phenylbenzimidazole-5 sulfonic acid and its salts, f) alkyl-ss, ss-diphenylacrylates for example alkyl a-cyano-ss, ss-diphenylacrylates such as octocrylene, g) triazines such as 2,4,6-trianilino- (p-carbo-2-ethyl-hexyl-1-oxi)-1, 3,5 triazine, h) camphor derivatives such as methylbenzylidene camphor and i) mixtures thereof. Other preferred sunscreen ingredients include those selected from the group consisting of: homosalate, Ethylhexyl salicylate, Diethylhexylbutamido triazone, Bis-ethylhexyloxyphenol methoxyphenyl triazine, Diethylamino hydroxybenzoyl hexyl benzoate, Butyl methoxydibenzoylmethane, Methylene bis-benzotriazoyl tetramethylbutylphenol, Polysilicone-15 and mixtures thereof. A sunscreen agent is optionally present in an amount from 0.1 to 10% by weight of the composition.

### Other Optional Ingredients

The compositions of the present invention may also optionally comprise one or more of the following optional ingredients. Preservatives may be added to the emulsion such as 2-bromo2-nitropropane-1,3-diol (bronopol, which is available commercially under the trade name Myacide RTM), benzyl alcohol, diazolidinyl urea, imidazolidinyl urea, methyl paraben, phenoxy ethanol, ethyl paraben, propyl paraben, sodium methyl paraben, sodium dehydroacetate, polyhexamethylenebiguanide hydrochloride, isothiazolone, sodium benzoate, chlorhexidine digluconate and sodium propyl paraben, suitably in an amount of from 0.01% to 10% by weight of the emulsion.

Thickeners, viscosity modifying agents and/or gelling agents may be added to the emulsion composition, such as acrylic acid polymers e. g. available commercially under the trade name Carbopol, Novethix, EZ 4U or Ultrez (Lubrizol) or Sepigel, Sepiplus and Simulgel (Seppic). Also modified cellloses e. g. hydroxyethylcellulose available commercially under the trade name Natrosol (Hercules) or hydroxypropylmethyl cellulose, amine oxides, block polymers of ethylene oxide and propylene oxide (for example, those available from BASF Wyandotte under the trade name"Pluronic"RTM), PVM, MA, or a decadiene crosspolymer (available under the trade name Stabilez 60), ethoxylated fatty alcohols, salt (magnesium chloride, sodium chloride), Aristoflex AVC (Clariant), phthalic acid amide, xanthan gum, sodium polyacrylate, polyvinyl alcohols, fatty alcohols and alkyl galactomannans available under the trade name N-Hance from Hercules, suitably in an amount of from 0.1% to 10% by weight of the composition.

Sequestering agents may be added to the emulsion composition, such as ethylenediamine tetraacetic acid and salts thereof, suitably in an amount of from 0.005% to 0.5% by weight of the composition.

The composition may also include waxes such as cocoa butter suitably in an amount of from 1% to 99% by weight of the composition.

The composition may also comprise suitable, cosmetically acceptable diluents, carriers and/or propellants such as dimethyl ether.

The composition may also include pearlising agents such as stearic monoethanolamide and/or mica, suitably in an amount of from 0.01% to 10% by weight of the composition.

Perfumes may be added suitably in an amount of from 0.01% to 2% by weight of the composition, as may water soluble dyes such as tartrazine, suitably in an amount of from a trace amount (such as 1 x 10-5 %) to 0.1 % by weight of the composition.

The composition may also include pH adjusting agents such as sodium hydroxide, aminomethyl propanol, triethanolamine, suitably in an amount of from 0.01 % to 10% by weight of the composition. The composition may be buffered by means well known in the art, for example by use of buffer systems comprising succinic acid, citric acid, lactic acid, and acceptable salts thereof, phosphoric acid, mono-or disodium phosphate and sodium carbonate. Suitably, the composition may have a pH between 3 and 10, preferably between 4 and 8.

### Method of use

The method may comprise administering a therapeutically effective amount of the composition comprising the matrikine. Administering may comprising contacting the composition with skin of the subject. Preferably, the administering comprises contacting the composition with facial skin of the subject.

The subject may be a mammal. Preferably, the subject is a human being.

The term *"comprising"* may refer to *"consisting of"* or *"consisting essentially of"*.

All of the embodiments and features described herein (including any accompanying claims, abstract and drawings), and/or all of the steps of any method or process so disclosed, may be combined with any of the above aspects or embodiments in any combination, unless stated otherwise with reference to a specific combination, for example, combinations where at least some of such features and/or steps are mutually exclusive.

For a better understanding of the invention, and to show embodiments of the invention may be put into effect, reference will now be made, by way of example, to the accompanying drawings, in which:-
Figure 1 is a schematic diagram showing a HSP sphere within a 3D HSP space;
Figure 2 is a HSP space showing a HSP sphere of a solute, which surrounded by and containing smaller HSP spheres of solvents;
Figure 3 is a HSP space showing a HSP sphere for Pal-GQPR (Matrixyl 3000) with (A) solvents scoring 1 and 2 inside its sphere, and (B) solvents scoring 1, 2 and 3 inside its sphere;
**Figure 4** is a HSP space showing a HSP sphere for Pal-QTAV with solvents scoring 1 and 2 inside its sphere;
**Figure 5** is a HSP space showing a HSP sphere for Idealift with (A) solvents scoring 1 inside its sphere, (B) solvents scoring 1 and 2 inside its sphere, and (C) solvents scoring 1, 2 and 3 inside its sphere;
**Figure 6** is a HSP space showing a HSP sphere for Pal-EKGD with solvents scoring 1 inside its sphere;
**Figure 7** is a HSP space showing a HSP sphere for Pal-GHK (Matrixyl 3000) with (A) solvents scoring 1 and 2 inside its sphere, and (B) solvents scoring 1, 2 and 3 inside its sphere;
**Figure 8** is a HSP space showing a HSP sphere for Pal-LSVD with (A) solvents scoring 1 inside its sphere, and (B) solvents scoring 1 and 2 inside its sphere; and
**Figure 9** is a HSP space showing a HSP sphere for Pal-GPKG with solvents scoring 1 inside its sphere.

### Examples

### 1. HSP space and HSP sphere

Figure 1 shows a HSP sphere of a material within a 3-dimensional HSP space defined by a δD axis, a δH axis and a δP axis. The HSP values of the material are defined by the location of the centre of the sphere.

Figure 2 is a HSP space showing a large HSP sphere of a test material (e.g., a matrikine). Inside the HSP sphere are smaller HSP spheres of reference solvents which have been found to dissolve the test material. Surrounding the HSP sphere of the test material are smaller HSP (shown as cubes) what do not dissolve the test material.

### 2. Determining the HSP sphere of several matrikines

The HSP sphere of seven different matrikines was determined by using a range of reference solvents (solvents with known HSP spheres) to dissolve the matrikines. The reference solvents were then ranked based on their ability to dissolve the matrikines. A software package called Hansen Solubility Parameters in Practice (HSPiP) was then used to determine the HSPs of each of the matrikines.

### Reference Materials

In general, a set of around 20 standard reference solvents was used to carry out preliminary investigations in solubility tests. Following this, additional/fine-tuning (FT) solvents were selected and used to are used to optimise the reliability and definition of the solubility parameters of the matrikines.

### Experimental conditions

All samples were prepared under standard conditions, within an air-conditioned laboratory environment, at 20°C and a standard pressure.

### Sample preparation

Usually, samples are prepared on the Formax High-throughput Formulation System, manufactured by Chemspeed. However due to the small sample size, the samples of this project were prepared manually using a single channel pipette for liquids and an analytical balance for solids.

### Qualitative Solubility

Qualitative evaluation of the solubility of the matrikines within a range of different reference solvents was used. Samples were placed into six categories, according to the relative extent to which the matrikine dissolved in the solvent.

Depending upon the category into which it has been placed, a sample was given a solubility ranking, between 1 and 6, where '1' indicates a sample with complete solubility and '6' a sample that is untouched by the solvent, exhibiting no dissolution effects whatsoever. Between these two extremes sit the other four ranking-categories, whose typical descriptions can be found in table 1 below.

**Table 1:**

| **Solubility Rank** | **Precipitate** | **Turbidity** |
|---|---|---|
| 1 | None | None |
| 2 | None | Weak |
| 3 | None | Strong |
| 4 | Trace | Strong |
| 5 | Moderate | Weak |
| 6 | Complete | None |

### HSPiP Analysis

The use of qualitative rankings of solubility phenomena makes it possible to define a spheroidal cluster in which the most compatible samples are contained. This cluster is called the Hansen Solubility Sphere and its central coordinates (in δD, dispersion forces, δP, polarity, and δH, hydrogen bonding) define the solubility parameters of the product itself.

The scores from 1 to 6 were entered into the HSPiP data form and then the software analyses the data to produce a Hansen diagram of the solubility sphere, alongside details of the solubility parameters and the goodness of the fit of the data to the sphere, in text format.

### Results - Raw data:

### Matrikine 1 - Pal-GQPR (Matrixyl 3000 (SEQ ID No. 62))

The solubility scores and the HSPs for the reference solvents that form the basis of the HSP analysis are given table 2 below.

**Table 2:**

| ***No*** | ***Solvent*** | ***D*** | ***P*** | ***H*** | ***MVol*** | ***Score*** |
|---|---|---|---|---|---|---|
| ACN | Acetonitrile | 15.3 | 18 | 6.1 | 52.9 | 5 |
| 1BrNp | 1-Bromonaphthalene | 20.6 | 3.1 | 4.1 | 140.1 | 3 |
| BuAc | n-Butyl Acetate | 15.8 | 3.7 | 6.3 | 132.6 | 5 |
| GBL | γ-Butyrolactone (Gbl) | 18 | 16.6 | 7.4 | 76.5 | 5 |
| CyHon | Cyclohexanone | 17.8 | 8.4 | 5.1 | 104.2 | 5 |
| CPS | Decamethylcy clopentasiloxane | 12.9 | 1.3 | 1 | 388.7 | 5 |
| DMF | Dimethyl Formamide (Dmf) | 17.4 | 13.7 | 11.3 | 77.4 | 5 |
| DMSO | Dimethyl Sulfoxide (Dmso) | 18.4 | 16.4 | 10.2 | 71.3 | 3 |
| 14Diox | 1,4-Dioxane | 17.5 | 1.8 | 9 | 85.7 | 5 |
| GC | Glycerol Carbonate | 17.9 | 25.5 | 17.4 | 83.2 | 6 |
| Hex | Hexane | 14.9 | 0 | 0 | 131.4 | 4 |
| MEK | Methyl Ethyl Ketone (Mek) | 16 | 9 | 5.1 | 90.2 | 5 |
| NMF | n-Methyl Formamide | 17.4 | 18.8 | 15.9 | 59.1 | 1 |
| NMP | n-Methyl-2-Pyrrolidone (Nmp) | 18 | 12.3 | 7.2 | 96.6 | 4 |
| 2Phet | 2-Phenoxy Ethanol | 17.8 | 5.7 | 14.3 | 124.7 | 2 |
| IPA | 2-Propanol | 15.8 | 6.1 | 16.4 | 76.9 | 6 |
| PC | Propylene Carbonate | 20 | 18 | 4.1 | 85.2 | 5 |
| PG | Propylene Glycol | 16.8 | 10.4 | 21.3 | 73.7 | 6 |
| PM | Propylene Glycol Monomethyl Ether | 15.6 | 6.3 | 11.6 | 98.2 | 5 |
| THF | Tetrahydrofur an (Thf) | 16.8 | 5.7 | 8 | 81.9 | 5 |
| TOL | Toluene | 18 | 1.4 | 2 | 106.6 | 3 |
| CIBz | Chlorobenzene | 19 | 4.3 | 2 | 102.1 | 4 |
| H2O | Water | 15.5 | 16 | 42.3 | 18 | 5 |
| 1 | Benzyl Alcohol:2-Propanol 20: 80% | 16.3 | 6.1 | 15.9 | 82.3 | 4 |
| 2 | Benzyl Alcohol:2-Propanol 40 : 60% | 16.8 | 6.2 | 15.3 | 87.7 | 4 |
| 3 | Benzyl Alcohol:2-Propanol 60 : 40% | 17.36 | 6.22 | 14.78 | 93.04 | 3 |
| 4 | Benzyl Alcohol:2-Propanol 80 : 20% | 17.88 | 6.26 | 14.24 | 98.42 | 3 |
| 5 | Glycerol Carbonate:Acetone 20 : 80% | 16 | 13.4 | 9.1 | 75.7 | 5 |
| 6 | Glycerol Carbonate:Acetone 40 : 60% | 16.5 | 16.4 | 11.2 | 77.6 | 5 |
| 7 | Glycerol Carbonate:Acetone 60 : 40% | 16.9 | 19.5 | 13.2 | 79.4 | 5 |
| 8 | Glycerol Carbonate:Acetone 80 : 20% | 17.42 | 22.48 | 15.32 | 81.32 | 2 |
| 9 | Glycerol Carbonate:Ethanol 20 : 80% | 16.2 | 12.1 | 19 | 63.5 | 6 |
| 10 | Glycerol Carbonate:Ethanol 40 : 60% | 16.6 | 15.5 | 18.6 | 68.4 | 6 |
| 11 | Glycerol Carbonate:Ethanol 60 : 40% | 17.1 | 18.8 | 18.2 | 73.4 | 6 |
| 12 | Glycerol Carbonate:Ethanol 80 : 20% | 17.48 | 22.16 | 17.8 | 78.28 | 6 |
| 13 | n-Methyl Formamide:Propylene Carbonate 20 : 80% | 19.5 | 18.2 | 6.5 | 80 | 5 |
| 14 | n-Methyl Formamide:Propylene Carbonate 40 : 60% | 19 | 18.3 | 8.8 | 74.8 | 5 |
| 15 | n-Methyl Formamide:Propylene Carbonate 60 : 40% | 18.44 | 18.48 | 11.18 | 69.54 | 4 |
| 16 | n-Methyl Formamide:Propylene Carbonate 80 : 20% | 17.92 | 18.64 | 13.54 | 64.32 | 6 |
| 17 | Dimethyl sulfoxide:Methanol 66:34% | 17.1 | 15 | 14.3 | 100 | 6 |
| 18 | Glycerol Carbonate: Dimethyl Formamide:1-Prop 55:25:20% | 17.4 | 18.8 | 15.9 | 100 | 5 |
| 19 | Glycerol :Dimethyl sulfoxide 28:72% | 18.1 | 15 | 15 | 100 | 6 |

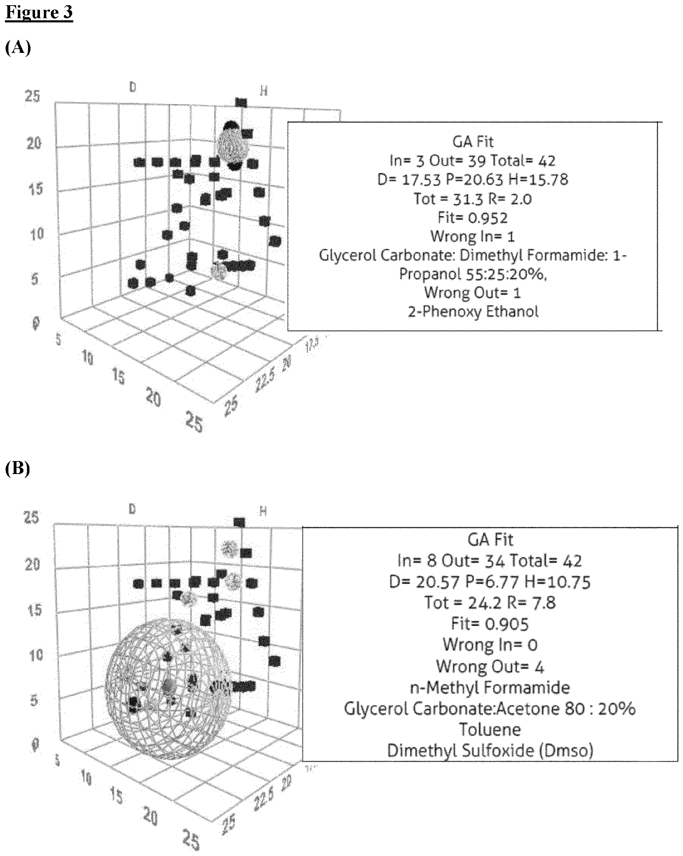

From the initial 21 solvents, only N-Methyl Formamide showed complete dissolution and 2-PhenoxyEthanol displayed the most interaction with the product without complete dissolution. These 2 areas of HSP space were further explored with the finetuning samples. No other samples displayed complete dissolution, but some showed varying levels of interaction. The HSPiP Software requires at least 2 solvents to be inside the sphere so the analysis had to be run on samples with a score of 1 & 2 inside of the sphere (see Figure 3).

The blend of Benzyl Alcohol:2-Propanol 80:20% gives a HSP value very close to 2-Phenoxy Ethanol and did not dissolve. The blend of Glycerol Carbonate: Dimethyl Formamide: 1-Propanol 55:25:20%, matches the HSP of N-Methyl Formamide and did not dissolve. Both of these results indicate that it might not be HSP dissolution that is seen in N-Methyl Formamide and 2-Phenoxyethanol, but interactions outside of HSP theory (i.e., reactions, molecule reorganisation, etc.).

When the samples that scored a 3 are also included in the analysis, it can be seen that the HSP sphere moves into a different area of HSP space: this indicates that this determination is not displaying the true HSP values of this material due to lack of dissolution. Therefore, this sphere cannot be trusted, though the information about the individual solvents may still be useful.

### Matrikine 2 - Pal-QTAV (SEQ ID No. 73)

The solubility scores and the HSPs for the reference solvents that form the basis of the HSP analysis are given in table 3 below.

**Table 3:**

| ***No*** | ***Solvent*** | ***D*** | ***P*** | ***H*** | ***MVol*** | ***Score*** |
|---|---|---|---|---|---|---|
| ACN | Acetonitrile | 15.3 | 18 | 6.1 | 52.9 | 4 |
| BrNp | 1-Bromonapht halene | 20.6 | 3.1 | 4.1 | 140.1 | 4 |
| BuAc | n-Butyl Acetate | 15.8 | 3.7 | 6.3 | 132.6 | 4 |
| GBL | γ-Butyrolactone (Gbl) | 18 | 16.6 | 7.4 | 76.5 | 4 |
| CyHon | Cyclohexanone | 17.8 | 8.4 | 5.1 | 104.2 | 4 |
| CPS | Decamethylcyclopentasiloxane | 12.9 | 1.3 | 1 | 388.7 | 4 |
| DMF | Dimethyl Formamide (Dmf) | 17.4 | 13.7 | 11.3 | 77.4 | 1 |
| DMSO | Dimethyl Sulfoxide (Dmso) | 18.4 | 16.4 | 10.2 | 71.3 | 1 |
| Diox | 1,4-Dioxane | 17.5 | 1.8 | 9 | 85.7 | 4 |
| GC | Glycerol Carbonate | 17.9 | 25.5 | 17.4 | 83.2 | 5 |
| Hex | Hexane | 14.9 | 0 | 0 | 131.4 | 4 |
| MEK | Methyl Ethyl Ketone (Mek) | 16 | 9 | 5.1 | 90.2 | 4 |
| NMF | n-Methyl Formamide (Nmf) | 17.4 | 18.8 | 15.9 | 59.1 | 1* |
| NMP | n-Methyl-2-Pyrrolidone (Nmp) | 18 | 12.3 | 7.2 | 96.6 | 1 |
| 2Phet | 2-Phenoxy Ethanol | 17.8 | 5.7 | 14.3 | 124.7 | 1 |
| IPA | 2-Propanol | 15.8 | 6.1 | 16.4 | 76.9 | 4 |
| PC | Propylene Carbonate | 20 | 18 | 4.1 | 85.2 | 4 |
| PG | Propylene Glycol | 16.8 | 10.4 | 21.3 | 73.7 | 2 |
| PM | Propylene Glycol Monomethyl Ether | 15.6 | 6.3 | 11.6 | 98.2 | 1 |
| THF | Tetrahydrofuran (Thf) | 16.8 | 5.7 | 8 | 81.9 | 1 |
| TOL | Toluene | 18 | 1.4 | 2 | 106.6 | 4 |
| BA | Benzyl Alcohol | 18.4 | 6.3 | 13.7 | 103.8 | 1 |
| BB | Benzyl Benzoate | 20 | 5.1 | 5.2 | 190.3 | 5 |
| Diac | Diacetone Alcohol | 15.8 | 8.2 | 10.8 | 124.3 | 1 |
| EtAc | Ethyl Acetate | 15.8 | 5.3 | 7.2 | 98.6 | 4 |
| 2EH | 2-Ethyl-Hexanol | 15.9 | 3.3 | 11.8 | 156.9 | 4 |
| TEC | Triethyl Citrate | 16.5 | 4.9 | 12 | 243 | 4 |
| 1 | 1,4-Dioxane:Dipr opylene Glycol:Methylal 39:35:26% | 16.5 | 4.9 | 11.9 | 103 | 3 |
| 2 | Glycerol Carbonate:Dimethyl Formamide:1-Propanol 55:25:20% | 17.4 | 18.8 | 15.9 | 80 | 2 |
| 3 | Glycerol Carbonate:2-Propanol 10 : 90% | 16.01 | 8.04 | 16.5 | 77.53 | 3 |
| 4 | Glycerol Carbonate:2-Propanol 20 : 80% | 16.22 | 9.98 | 16.6 | 78.16 | 3 |
| 5 | Glycerol Carbonate:2-Propanol 30 : 70% | 16.43 | 11.92 | 16.7 | 78.79 | 6 |
| 6 | Glycerol Carbonate:2-Propanol 40 : 60% | 16.64 | 13.86 | 16.8 | 79.42 | 6 |

| | | | | | | |
|---|---|---|---|---|---|---|
| *NMF was removed from the data set as it is believed to be an outlier | | | | | | |

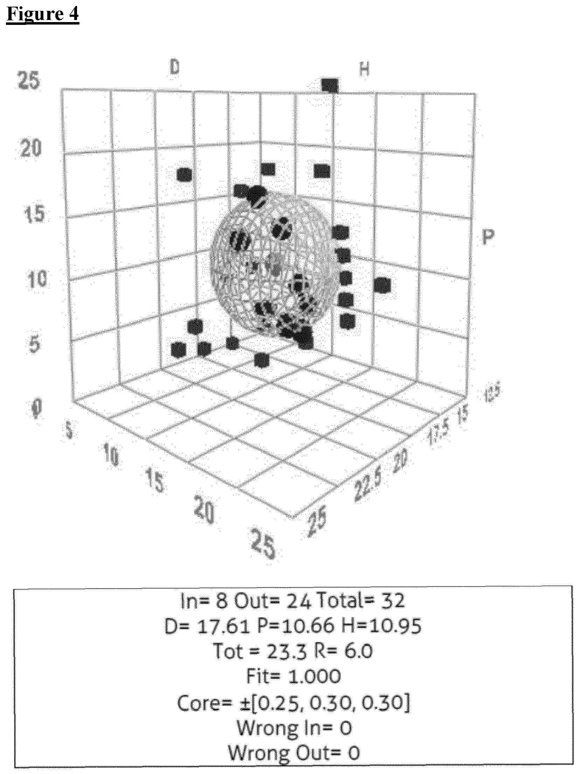

Pal-QTAV has a moderate δD value, a moderate δP value and a low δH value. Dispersion forces provide the greatest contribution to the cohesive energy density of Pal-QTAV. The product has a radius of 6 units. The experimental data has a fit score of 1 which is a perfect score (see Figure 4).

NMF was removed from this data set, because it is thought that a reaction was occurring outside of HSP. A blend of Glycerol Carbonate:Dimethyl Formamide: 1-Propanol 55:25:20% was made (it has similar HSP values to NMF): this sample didn't dissolve. This confirms that NMF should be removed from the set.

### Matrikine 3 - Idealift

The solubility scores and the HSPs for the reference solvents that form the basis of the HSP analysis are given in table 4 below.

**Table 4:**

| ***No*** | ***Solvent*** | ***D*** | ***P*** | ***H*** | ***MVol*** | ***Score*** |
|---|---|---|---|---|---|---|
| ACN | Acetonitrile | 15.3 | 18 | 6.1 | 52.9 | 6 |
| BrNp | 1-Bromonaphth alene | 20.6 | 3.1 | 4.1 | 140.1 | 6 |
| BuAc | n-Butyl Acetate | 15.8 | 3.7 | 6.3 | 132.6 | 5 |
| GBL | γ-Butyrolactone (Gbl) | 18 | 16.6 | 7.4 | 76.5 | 4 |
| CyHon | Cyclohexanone | 17.8 | 8.4 | 5.1 | 104.2 | 4 |
| CPS | Decamethylc yclopentasiloxane | 12.9 | 1.3 | 1 | 388.7 | 6 |
| DMF | Dimethyl Formamide (Dmf) | 17.4 | 13.7 | 11.3 | 77.4 | 3 |
| DMSO | Dimethyl Sulfoxide (Dmso) | 18.4 | 16.4 | 10.2 | 71.3 | 1 |
| 14Diox | 1,4-Dioxane | 17.5 | 1.8 | 9 | 85.7 | 4 |
| GC | Glycerol Carbonate | 17.9 | 25.5 | 17.4 | 83.2 | 3 |
| Hex | Hexane | 14.9 | 0 | 0 | 131.4 | 5 |
| MEK | Methyl Ethyl Ketone (Mek) | 16 | 9 | 5.1 | 90.2 | 4 |
| NMF | n-Methyl Formamide (Nmf) | 17.4 | 18.8 | 15.9 | 59.1 | 2 |
| NMP | n-Methyl-2-Pyrrolidone (Nmp) | 18 | 12.3 | 7.2 | 96.6 | 1 |
| 2Phet | 2-Phenoxy Ethanol | 17.8 | 5.7 | 14.3 | 124.7 | 2 |
| IPA | 2-Propanol | 15.8 | 6.1 | 16.4 | 76.9 | 4 |
| PC | Propylene Carbonate | 20 | 18 | 4.1 | 85.2 | 5 |
| PG | Propylene Glycol | 16.8 | 10.4 | 21.3 | 73.7 | 4 |
| PM | Propylene Glycol Monomethyl Ether | 15.6 | 6.3 | 11.6 | 98.2 | 4 |
| THF | Tetrahydrofuran (Thf) | 16.8 | 5.7 | 8 | 81.9 | 4 |
| TOL | Toluene | 18 | 1.4 | 2 | 106.6 | 6 |
| ACO | Acetone | 15.5 | 10.4 | 7 | 73.8 | 4 |
| BA | Benzyl Alcohol | 18.4 | 6.3 | 13.7 | 103.8 | 3 |
| BB | Benzyl Benzoate | 20 | 5.1 | 5.2 | 190.3 | 5 |
| CyHex | Cyclohexane | 16.8 | 0 | 0.2 | 108.9 | 5 |
| 13Diox | 1,3-Dioxolane | 18.1 | 6.6 | 9.3 | 69.9 | 4 |
| Xyl | p-Xylene | 17.8 | 1 | 3.1 | 121.1 | 5 |
| 4 | γ-Butyrolactone (Gbl):1,4-Dioxane 40: 60% | 17.7 | 7.7 | 8.4 | 82 | 5 |
| 5 | γ-Butyrolactone (Gbl):1,4-Dioxane 50: 50% | 17.75 | 9.2 | 8.2 | 81.1 | 4 |
| 6 | γ-Butyrolactone (Gbl):1,4-Dioxane 60: 40% | 17.8 | 10.68 | 8.04 | 80.18 | 3 |
| 7 | γ-Butyrolactone (Gbl):1,4-Dioxane 70: 30% | 17.85 | 12.16 | 7.88 | 79.26 | 3 |
| 8 | γ-Butyrolactone (Gbl):1,4-Dioxane 80: 20% | 17.9 | 13.64 | 7.72 | 78.34 | 3 |
| 9 | γ-Butyrolactone (Gbl):1,4-Dioxane 90: 10% | 17.95 | 15.12 | 7.56 | 77.42 | 3 |
| 10 | Glycerol Carbonate:Di methyl Formamide (Dmf) 20 : 80% | 17.5 | 16.1 | 12.5 | 78.6 | 3 |
| 11 | Glycerol Carbonate:Di methyl | 17.6 | 18.4 | 13.7 | 79.7 | 3 |
| | Formamide (Dmf) 40 : 60% | | | | | |
| 12 | Glycerol Carbonate:Di methyl | 17.7 | 20.8 | 15 | 80.9 | 3 |
| | Formamide (Dmf) 60 : 40% | | | | | |
| 13 | Glycerol Carbonate:Di methyl | 17.8 | 23.14 | 16.18 | 82.04 | 3 |
| | Formamide (Dmf) 80 : 20% | | | | | |
| 14 | n-Methyl Formamide:Acetonitrile 70 : 30% | 16.77 | 18.56 | 12.96 | 57.24 | 3 |
| 15 | n-Methyl-2-Pyrrolidone (Nmp):Glycer ol Carbonate 70 : 30% | 17.97 | 16.26 | 10.26 | 92.58 | 3 |
| 16 | n-Methyl-2-Pyrrolidone : N-Methyl Formamide : Acetonitrile 55:43:2% | 17.7 | 15.2 | 10.9 | 100 | 3 |
| 17 | Propylene Carbonate:Benzyl Alcohol 30 : 70% | 18.88 | 9.81 | 10.82 | 98.22 | 3 |
| 18 | Propylene Carbonate:Benzyl Alcohol 40 : 60% | 19.04 | 10.98 | 9.86 | 96.36 | 3 |
| 19 | Propylene Carbonate:Benzyl Alcohol 50 : 50% | 19.2 | 12.15 | 8.9 | 94.5 | 3 |
| 20 | Propylene Carbonate:Benzyl Alcohol 60 : 40% | 19.36 | 13.32 | 7.94 | 92.64 | 3 |
| 21 | Propylene Carbonate:Benzyl Alcohol 70 : 30% | 19.52 | 14.49 | 6.98 | 90.78 | 3 |
| 22 | Propylene Carbonate:Benzyl Alcohol 80 : 20% | 19.68 | 15.66 | 6.02 | 88.92 | 3 |
| 23 | Propylene Carbonate: Pr opylene Glycol Monomethyl Ether 20 : 80% | 16.5 | 8.6 | 10.1 | 95.6 | 3 |
| 24 | Propylene Carbonate: Pr opylene Glycol Monomethyl Ether 40 : 60% | 17.4 | 11 | 8.6 | 93 | 3 |
| 25 | Propylene Carbonate: Pr opylene Glycol Monomethyl Ether 50 : 50% | 17.8 | 12.15 | 7.85 | 91.7 | 3 |
| 26 | Propylene Carbonate: Pr opylene Glycol Monomethyl Ether 60 : 40% | 18.2 | 13.3 | 7.1 | 90.4 | 3 |
| 27 | Propylene Carbonate: Pr opylene Glycol Monomethyl Ether 80 : 20% | 19.12 | 15.66 | 5.6 | 87.8 | 3 |

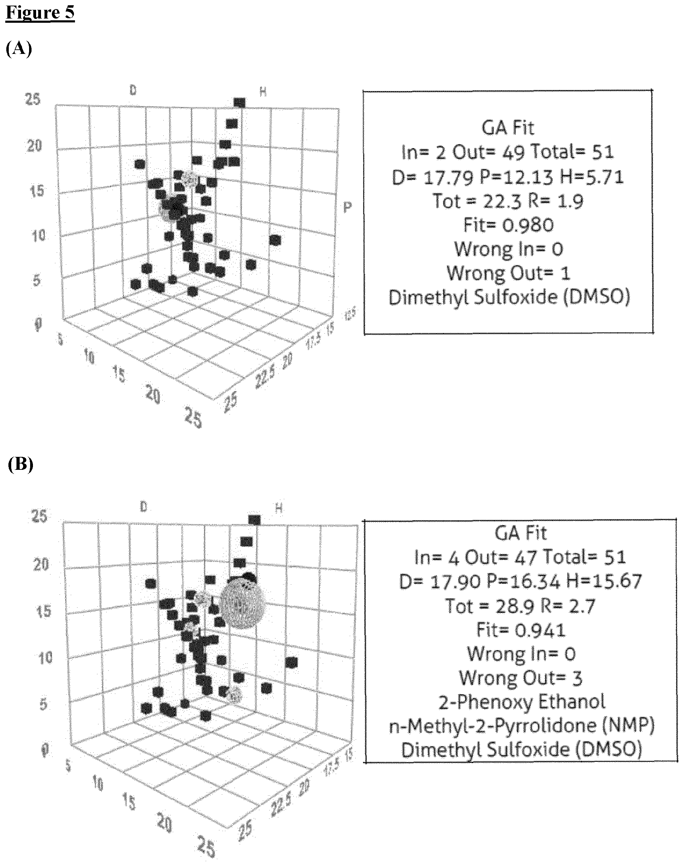

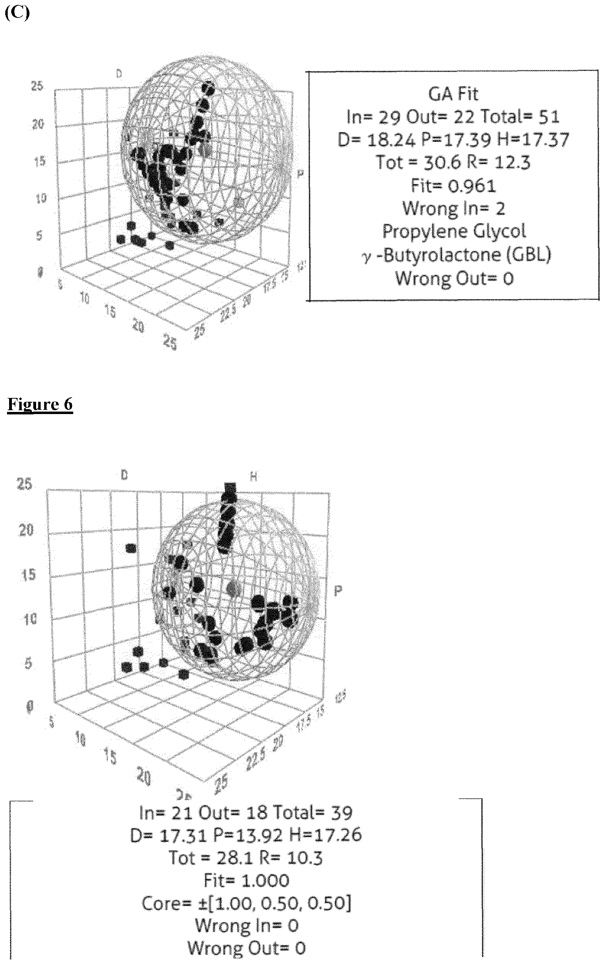

Only DMSO and NMP showed complete dissolution, but there are lots of reference solvents close by these solvents HSP that do not dissolve, indicating that these are outliers. Similar results were seen for those samples with scores of a 2. So, the analysis was extended to include samples with a 1, 2 and 3 inside. This analysis is based upon swelling and partial dissolution rather than pure dissolution (see Figure 5).

Idealift has a high δD value, a moderate δP value and a moderate δH value. Dispersion forces provide the greatest contribution to the cohesive energy density of Idealift. The product has a radius of 12.3 units. The experimental data has a fit score of 0.961. As this product does not have complete dissolution it is possible that other mechanisms might be needed when formulating with the product such as surfactants.

### Matrikine 4 - Pal-EKGD (SEQ ID No. 74)

The solubility scores and the HSPs for the reference solvents that form the basis of the HSP analysis are given in table 5 below.

**Table 5:**

| ***No*** | ***Solvent*** | ***D*** | ***P*** | ***H*** | ***MVol*** | ***Score*** |
|---|---|---|---|---|---|---|
| ACN | Acetonitrile | 15.3 | 18 | 6.1 | 52.9 | 5 |
| BrNp | 1-Bromonaphthalene | 20.6 | 3.1 | 4.1 | 140.1 | 4 |
| BuAc | n-Butyl Acetate | 15.8 | 3.7 | 6.3 | 132.6 | 4 |
| GBL | γ-Butyrolactone (Gbl) | 18 | 16.6 | 7.4 | 76.5 | 4 |
| CyHon | Cyclohexanone | 17.8 | 8.4 | 5.1 | 104.2 | 4 |
| CPS | Decamethylcyclopentasiloxane | 12.9 | 1.3 | 1 | 388.7 | 4 |
| DMF | Dimethyl Formamide (Dmf) | 17.4 | 13.7 | 11.3 | 77.4 | 1 |
| DMSO | Dimethyl Sulfoxide (Dmso) | 18.4 | 16.4 | 10.2 | 71.3 | 1 |
| Diox | 1,4-Dioxane | 17.5 | 1.8 | 9 | 85.7 | 6 |
| GC | Glycerol Carbonate | 17.9 | 25.5 | 17.4 | 83.2 | 3 |
| Hex | Hexane | 14.9 | 0 | 0 | 131.4 | 4 |
| MEK | Methyl Ethyl Ketone (Mek) | 16 | 9 | 5.1 | 90.2 | 4 |
| NMF | n-Methyl Formamide (Nmf) | 17.4 | 18.8 | 15.9 | 59.1 | 1 |
| NMP | n-Methyl-2-Pyrrolidone (Nmp) | 18 | 12.3 | 7.2 | 96.6 | 1 |
| 2Phet | 2-Phenoxy Ethanol | 17.8 | 5.7 | 14.3 | 124.7 | 1 |
| IPA | 2-Propanol | 15.8 | 6.1 | 16.4 | 76.9 | 1 |
| PC | Propylene Carbonate | 20 | 18 | 4.1 | 85.2 | 3 |
| PG | Propylene Glycol | 16.8 | 10.4 | 21.3 | 73.7 | 1 |
| PM | Propylene Glycol Monomethyl Ether | 15.6 | 6.3 | 11.6 | 98.2 | 1 |
| THF | Tetrahydrofuran (Thf) | 16.8 | 5.7 | 8 | 81.9 | 5 |
| TOL | Toluene | 18 | 1.4 | 2 | 106.6 | 3 |
| ACO | Acetone | 15.5 | 10.4 | 7 | 73.8 | 6 |
| BA | Benzyl Alcohol | 18.4 | 6.3 | 13.7 | 103.8 | 1 |
| BB | Benzyl Benzoate | 20 | 5.1 | 5.2 | 190.3 | 4 |
| 13BD | 1,3-Butanediol | 16.5 | 8.1 | 20.9 | 90 | 1 |
| Diac | Diacetone Alcohol | 15.8 | 8.2 | 10.8 | 124.3 | 2 |
| EtOH | Ethanol | 15.8 | 8.8 | 19.4 | 58.6 | 1 |
| EtFO | Ethyl Formate | 15.5 | 8.4 | 8.4 | 80.9 | 6 |
| 2EH | 2-Ethyl-Hexanol | 15.9 | 3.3 | 11.8 | 156.9 | 4 |
| MeOH | Methanol | 14.7 | 12.3 | 22.3 | 40.6 | 1 |
| 1Prop | 1-Propanol | 16 | 6.8 | 17.4 | 75.1 | 1 |
| TEC | Triethyl Citrate | 16.5 | 4.9 | 12 | 243 | 4 |
| 1 | Glycerol:γ-Butyrolactone (Gbl) 70 : 30% | 17.58 | 12.89 | 21.26 | 74.33 | 1 |
| 2 | Glycerol:γ-Butyrolactone (Gbl) 80 : 20% | 17.52 | 12.36 | 23.24 | 74.02 | 1 |
| 3 | Glycerol:γ-Butyrolactone (Gbl) 90 : 10% | 17.46 | 11.83 | 25.22 | 73.71 | 1 |
| 4 | n-Methyl Formamide: Glycerol Carbonate 20 : 80% | 17.8 | 24.16 | 17.1 | 78.38 | 2 |
| 5 | n-Methyl Formamide: Glycerol Carbonate 40 : 60% | 17.7 | 22.82 | 16.8 | 73.56 | 2 |
| 6 | n-Methyl Formamide: Glycerol Carbonate 60 : 40% | 17.6 | 21.48 | 16.5 | 68.74 | 1 |
| 7 | n-Methyl Formamide: Glycerol Carbonate 80 : 20% | 17.5 | 20.14 | 16.2 | 63.92 | 1 |

Pal-EKGD has a moderate δD value, a moderate δP value and a moderate δH value. Dispersion forces provide the greatest contribution to the cohesive energy density of Pal-EKGD. The product has a radius of 10.3 units. The experimental data has a fit score of 1 which is a perfect score (see Figure 6).

### Matrikine 5 -Pal-GHK (Matrixyl 3000 (SEQ ID No. 75))

The solubility scores and the HSPs for the reference solvents that form the basis of the HSP analysis are given in table 6 below.

**Table 6:**

| ***No*** | ***Solvent*** | ***D*** | ***P*** | ***H*** | ***MVol*** | ***Score*** |
|---|---|---|---|---|---|---|
| ACN | Acetonitrile | 15.3 | 18 | 6.1 | 52.9 | 6 |
| BrNp | 1-Bromonapht halene | 20.6 | 3.1 | 4.1 | 140.1 | 4 |
| nBUAC | n-Butyl Acetate | 15.8 | 3.7 | 6.3 | 132.6 | 5 |
| GBL | γ-Butyrolactone (Gbl) | 18 | 16.6 | 7.4 | 76.5 | 5 |
| CyHON | Cyclohexanone | 17.8 | 8.4 | 5.1 | 104.2 | 5 |
| CPS | Decamethylc yclopentasiloxane | 12.9 | 1.3 | 1 | 388.7 | 6 |
| DMF | Dimethyl Formamide (Dmf) | 17.4 | 13.7 | 11.3 | 77.4 | 6 |
| DMSO | Dimethyl Sulfoxide (Dmso) | 18.4 | 16.4 | 10.2 | 71.3 | 3 |
| DIOX | 1,4-Dioxane | 17.5 | 1.8 | 9 | 85.7 | 5 |
| GC | Glycerol Carbonate | 17.9 | 25.5 | 17.4 | 83.2 | 5 |
| HEX | Hexane | 14.9 | 0 | 0 | 131.4 | 5 |
| MEK | Methyl Ethyl Ketone (Mek) | 16 | 9 | 5.1 | 90.2 | 5 |
| NMF | n-Methyl Formamide | 17.4 | 18.8 | 15.9 | 59.1 | 5 |
| NMP | n-Methyl-2- Pyrrolidone (Nmp) | 18 | 12.3 | 7.2 | 96.6 | 5 |
| 2PHET | 2-Phenoxy Ethanol | 17.8 | 5.7 | 14.3 | 124.7 | 2 |
| IPA | 2-Propanol | 15.8 | 6.1 | 16.4 | 76.9 | 5 |
| PC | Propylene Carbonate | 20 | 18 | 4.1 | 85.2 | 5 |
| PG | Propylene Glycol | 16.8 | 10.4 | 21.3 | 73.7 | 5 |
| PM | Propylene Glycol Monomethyl Ether | 15.6 | 6.3 | 11.6 | 98.2 | 5 |
| THF | Tetrahydrofuran (Thf) | 16.8 | 5.7 | 8 | 81.9 | 6 |
| TOL | Toluene | 18 | 1.4 | 2 | 106.6 | 4 |
| BA | Benzyl Alcohol | 18.4 | 6.3 | 13.7 | 103.8 | 2 |
| 2-But | 2-Butanol | 15.8 | 5.7 | 14.5 | 92 | 5 |
| CB | Chlorobenzene | 19 | 4.3 | 2 | 102.1 | 2 |
| CyHol | Cyclohexanol | 17.4 | 4.1 | 13.5 | 105.7 | 3 |
| 1Hex | 1-Hexanol | 15.9 | 5.8 | 12.5 | 125.2 | 6 |
| TEC | Triethyl Citrate | 16.5 | 4.9 | 12 | 243 | 3 |
| 1 | Benzyl Alcohol:2-Propanol 20 : 80% | 16.3 | 6.1 | 15.9 | 82.3 | 4 |
| 2 | Benzyl Alcohol:2-Propanol 40 : 60% | 16.8 | 6.2 | 15.3 | 87.7 | 4 |
| 3 | Benzyl Alcohol:2-Propanol 60 : 40% | 17.4 | 6.2 | 14.8 | 93 | 4 |
| 4 | Benzyl Alcohol:2-Propanol 80 : 20% | 17.88 | 6.26 | 14.24 | 98.42 | 3 |
| 5 | Cyclohexanone:2-Phenoxy | 17.8 | 6.2 | 12.5 | 120.6 | 3 |
| | Ethanol 20 : 80% | | | | | |
| 6 | Cyclohexanone:2-Phenoxy Ethanol 40 : 60% | 17.8 | 6.8 | 10.6 | 116.5 | 3 |
| 7 | Cyclohexanone:2-Phenoxy | 17.8 | 7.32 | 8.78 | 112.4 | 3 |
| | Ethanol 60 : 40% | | | | | |
| 8 | Cyclohexanone:2-Phenoxy | 17.8 | 7.86 | 6.94 | 108.3 | 3 |
| | Ethanol 80 : 20% | | | | | |

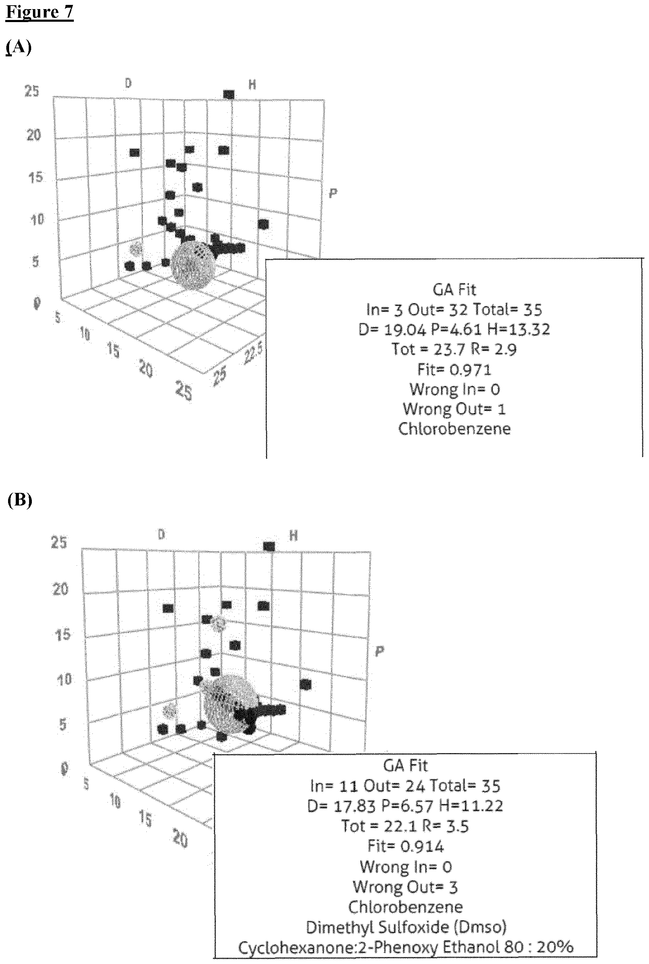

No samples showed complete dissolution, so the determination was based upon partial dissolution and swelling of the samples that scored a 2. This gives a small sphere with 1 solvent outlier. Due to the limited number of samples inside this sphere, the analysis was also extended to include samples with a 1, 2 and 3 inside: this second sphere shows further interactions in the same area of HSP space (see Figure 7).

Pal-GHK (SEQ ID No. 75) has a moderate δD value, a low δP value and a low δH value. Dispersion forces provide the greatest contribution to the cohesive energy density of Pal-GHK. The product has a radius of 3.5 units. The experimental data has a fit score of 0.914 against the single sphere model. As this product does not have complete dissolution it is possible that other mechanisms might be needed when formulating with the product such as surfactants.

### Matrikine 6- Pal-LSVD (SEQ ID No. 55)

The solubility scores and the HSPs for the reference solvents that form the basis of the HSP analysis are given in table 7 below.

| Table 7: | | | | | | |
|---|---|---|---|---|---|---|
| ***No*** | ***Solvent*** | ***D*** | ***P*** | ***H*** | ***MVol*** | ***Score*** |
| ACN | Acetonitrile | 15.3 | 18 | 6.1 | 52.9 | 5 |
| BRNP | 1-Bromonapht halene | 20.6 | 3.1 | 4.1 | 140.1 | 6 |
| BuAc | n-Butyl Acetate | 15.8 | 3.7 | 6.3 | 132.6 | 5 |
| GBL | γ-Butyrolactone (Gbl) | 18 | 16.6 | 7.4 | 76.5 | 6 |
| CyHon | Cyclohexanone | 17.8 | 8.4 | 5.1 | 104.2 | 5 |
| CPS | Decamethylcyclopentasiloxane | 12.9 | 1.3 | 1 | 388.7 | 6 |
| DMF | Dimethyl Formamide (Dmf) | 17.4 | 13.7 | 11.3 | 77.4 | 1 |
| DMSO | Dimethyl Sulfoxide (Dmso) | 18.4 | 16.4 | 10.2 | 71.3 | 1 |
| 14Diox | 1,4-Dioxane | 17.5 | 1.8 | 9 | 85.7 | 4 |
| GC | Glycerol Carbonate | 17.9 | 25.5 | 17.4 | 83.2 | 3 |
| Hex | Hexane | 14.9 | 0 | 0 | 131.4 | 3 |
| Mek | Methyl Ethyl Ketone (Mek) | 16 | 9 | 5.1 | 90.2 | 5 |
| NMF | n-Methyl Formamide (Nmf) | 17.4 | 18.8 | 15.9 | 59.1 | 3 |
| NMP | n-Methyl-2-Pyrrolidone (Nmp) | 18 | 12.3 | 7.2 | 96.6 | 1 |
| 2Phet | 2-Phenoxy | 17.8 | 5.7 | 14.3 | 124.7 | 2 |
| IPA | Ethanol 2-Propanol | 15.8 | 6.1 | 16.4 | 76.9 | 3 |
| PC | Propylene Carbonate | 20 | 18 | 4.1 | 85.2 | 4 |
| PG | Propylene Glycol | 16.8 | 10.4 | 21.3 | 73.7 | 3 |
| PM | Propylene Glycol Monomethyl Ether | 15.6 | 6.3 | 11.6 | 98.2 | 3 |
| THF | Tetrahydrofuran (Thf) | 16.8 | 5.7 | 8 | 81.9 | 3 |
| TOL | Toluene | 18 | 1.4 | 2 | 106.6 | 3 |
| ACO | Acetone | 15.5 | 10.4 | 7 | 73.8 | 3 |
| BA | Benzyl Alcohol | 18.4 | 6.3 | 13.7 | 103.8 | 2 |
| Diac | Diacetone Alcohol | 15.8 | 8.2 | 10.8 | 124.3 | 3 |
| 13Diox | 1,3-Dioxolane | 18.1 | 6.6 | 9.3 | 69.9 | 3 |
| EtFo | Ethyl Formate | 15.5 | 8.4 | 8.4 | 80.9 | 3 |
| Pyr | Pyridine | 19 | 8.8 | 5.9 | 80.9 | 1 |
| 1 | Acetonitrile:Benzyl Alcohol 20 : 80% | 17.8 | 8.6 | 12.2 | 93.6 | 2 |
| 2 | Acetonitrile:Benzyl Alcohol 40 : 60% | 17.2 | 11 | 10.7 | 83.4 | 2 |
| 3 | Acetonitrile:Benzyl Alcohol 60 : 40% | 16.5 | 13.3 | 9.1 | 73.3 | 3 |
| 4 | Acetonitrile:Benzyl Alcohol 80 : 20% | 15.92 | 15.66 | 7.62 | 63.08 | 4 |
| 5 | γ-Butyrolacton e | 18.3 | 8.4 | 12.4 | 98.3 | 2 |
| | (Gbl):Benzyl Alcohol 20 : 80% | | | | | |
| 6 | γ-Butyrolactone (Gbl):Benzyl Alcohol 40 : 60% | 18.2 | 10.4 | 11.2 | 92.9 | 2 |
| 7 | γ-Butyrolactone (Gbl):Benzyl Alcohol 60 : 40% | 18.2 | 12.5 | 9.9 | 87.4 | 4 |
| 8 | γ-Butyrolactone (Gbl):Benzyl Alcohol 80 : 20% | 18.08 | 14.54 | 8.66 | 81.96 | 4 |
| 9 | Cyclohexanone:N-Methyl Formamide 20 : 80% | 17.5 | 16.7 | 13.7 | 68.1 | 2 |
| 10 | Cyclohexanone:N-Methyl Formamide 40 : 60% | 17.6 | 14.6 | 11.6 | 77.1 | 2 |
| 11 | Cyclohexanone:N-Methyl Formamide 60 : 40% | 17.64 | 12.56 | 9.42 | 86.16 | 2 |
| 12 | Cyclohexanone:N-Methyl Formamide 80 : 20% | 17.72 | 10.48 | 7.26 | 95.18 | 3 |
| 13 | Propylene Carbonate:1, 3-Dioxolane 20 : 80% | 18.5 | 8.9 | 8.3 | 73 | 6 |
| 14 | Propylene Carbonate:1, 3-Dioxolane 40 : 60% | 18.9 | 11.2 | 7.2 | 76 | 6 |
| 15 | Propylene Carbonate:1, 3-Dioxolane 60 : 40% | 19.24 | 13.44 | 6.18 | 79.08 | 5 |
| 16 | Propylene Carbonate:1, 3-Dioxolane 80 : 20% | 19.6 | 15.7 | 5.1 | 82.1 | 6 |

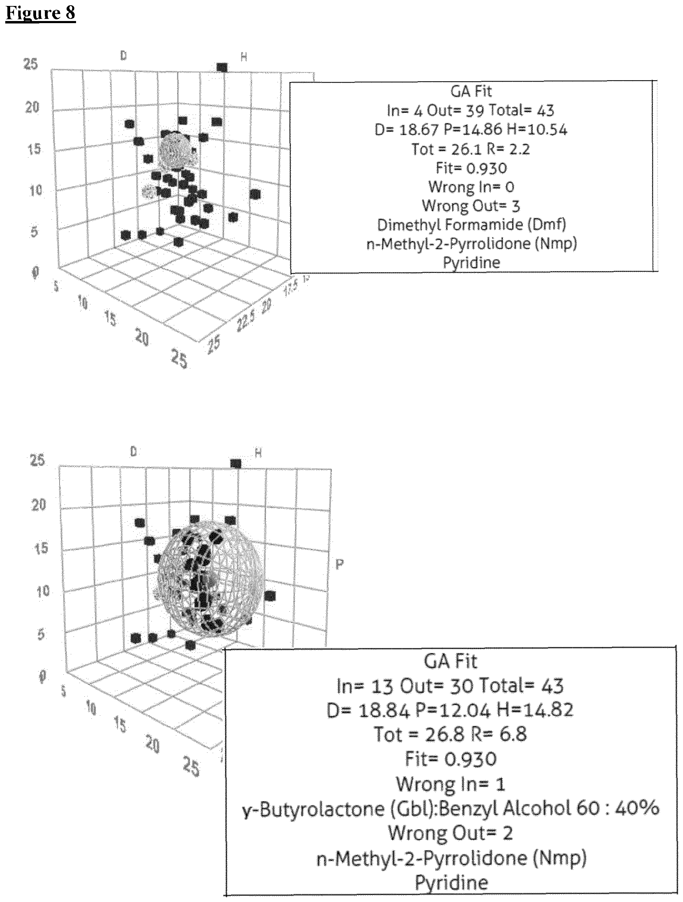

Pal-LSVD has very limited solubility but 4 samples did show complete solubility. These samples however do not make a sphere, so the analysis was run again with the samples that scored 1 and 2 inside the sphere. This second analysis makes a more coherent sphere. The second analysis is based upon swelling and partial dissolution rather than pure dissolution (see Figure 8).

Pal-LSVD has a high δD value, a moderate δP value and a low δH value. Dispersion forces provide the greatest contribution to the cohesive energy density of Pal-LSVD. The product has a radius of 6.8 units. The experimental data has a fit score of 0.93. As this product does not have complete dissolution it is possible that other mechanisms might be needed when formulating with the product such as surfactants.

### Matrikine 7 -Pal-GPKG (SEQ ID No. 76)

The solubility scores and the HSPs for the reference solvents that form the basis of the HSP analysis are given in table 8 below.

**Table 8:**

| ***No*** | ***Solvent*** | ***D*** | ***P*** | ***H*** | ***MVol*** | ***Score*** |
|---|---|---|---|---|---|---|
| ACN | Acetonitrile | 15.3 | 18 | 6.1 | 52.9 | 6 |
| BrNp | 1-Bromonaphthalene | 20.6 | 3.1 | 4.1 | 140.1 | 4 |
| BuAc | n-Butyl Acetate | 15.8 | 3.7 | 6.3 | 132.6 | 6 |
| GBL | γ-Butyrolactone (Gbl) | 18 | 16.6 | 7.4 | 76.5 | 5 |
| GyHon | Cyclohexanone | 17.8 | 8.4 | 5.1 | 104.2 | 6 |
| CPS | Decamethylcyclopentasiloxane | 12.9 | 1.3 | 1 | 388.7 | 6 |
| DMF | Dimethyl Formamide (Dmf) | 17.4 | 13.7 | 11.3 | 77.4 | 1 |
| DMSO | Dimethyl Sulfoxide (Dmso) | 18.4 | 16.4 | 10.2 | 71.3 | *1 |
| 14Diox | 1,4-Dioxane | 17.5 | 1.8 | 9 | 85.7 | 5 |
| GC | Glycerol Carbonate | 17.9 | 25.5 | 17.4 | 83.2 | 4 |
| Hex | Hexane | 14.9 | 0 | 0 | 131.4 | 6 |
| MEK | Methyl Ethyl Ketone (Mek) | 16 | 9 | 5.1 | 90.2 | 6 |
| NMF | n-Methyl Formamide (Nmf) | 17.4 | 18.8 | 15.9 | 59.1 | 2 |
| NMP | n-Methyl-2-Pyrrolidone (Nmp) | 18 | 12.3 | 7.2 | 96.6 | *1 |
| 2Pher | 2-Phenoxy Ethanol | 17.8 | 5.7 | 14.3 | 124.7 | 1 |
| IPA | 2-Propanol | 15.8 | 6.1 | 16.4 | 76.9 | 5 |
| PC | Propylene Carbonate | 20 | 18 | 4.1 | 85.2 | 5 |
| PG | Propylene Glycol | 16.8 | 10.4 | 21.3 | 73.7 | 1 |
| PM | Propylene Glycol Monomethyl Ether | 15.6 | 6.3 | 11.6 | 98.2 | 5 |
| THF | Tetrahydrofu ran (Thf) | 16.8 | 5.7 | 8 | 81.9 | 5 |
| Tol | Toluene | 18 | 1.4 | 2 | 106.6 | 3 |
| Aco BA | Acetone Benzyl Alcohol | 15.5 18.4 | 10.4 6.3 | 7 13.7 | 73.8 103.8 | 4 1 |
| BB | Benzyl Benzoate | 20 | 5.1 | 5.2 | 190.3 | 2 |
| 13BD | 1,3-Butanediol | 16.5 | 8.1 | 20.9 | 90 | 4 |
| Diac | Diacetone Alcohol | 15.8 | 8.2 | 10.8 | 124.3 | 5 |
| 13Diox | 1,3-Dioxolane | 18.1 | 6.6 | 9.3 | 69.9 | 6 |
| EtOH | Ethanol | 15.8 | 8.8 | 19.4 | 58.6 | 1 |
| EtFo | Ethyl Formate | 15.5 | 8.4 | 8.4 | 80.9 | 4 |
| FooH | Formic Acid | 14.6 | 10 | 14 | 37.9 | 1 |
| MEOH | Methanol | 14.7 | 12.3 | 22.3 | 40.6 | 1 |
| 1Prop | 1-Propanol | 16 | 6.8 | 17.4 | 75.1 | 4 |
| TEC | Triethyl Citrate | 16.5 | 4.9 | 12 | 243 | 2 |
| H2O | Water | 15.5 | 16 | 42.3 | 18 | 3 |
| 1 | γ-Butyrolactone (Gbl):Dimeth yl Formamide (Dmf) 20 : 80% | 17.5 | 14.3 | 10.5 | 77.2 | 1 |
| 2 | γ- Butyrolactone (Gbl):Dimethyl Formamide (Dmf) 40 : 60% | 17.6 | 14.9 | 9.7 | 77 | 5 |
| 3 | γ-Butyrolactone (Gbl):Dimeth yl Formamide (Dmf) 60 : 40% | 17.76 | 15.44 | 8.96 | 76.86 | 5 |
| 4 | γ-Butyrolactone (Gbl):Dimethyl Formamide (Dmf) 80 : 20% | 17.88 | 16.02 | 8.18 | 76.68 | 5 |
| 5 | Glycerol:Ethanol 20 : 80% | 16.1 | 9.3 | 21 | 61.6 | 1 |
| 6 | Glycerol:Ethanol 40 : 60% | 16.4 | 9.8 | 22.5 | 64.5 | 1 |
| 7 | Glycerol:Ethanol 60 : 40% | 16.8 | 10.3 | 24.1 | 67.5 | 1 |
| 8 | Glycerol:Ethanol 80 : 20% | 17.08 | 10.8 | 25.64 | 70.44 | 1 |
| 9 | n-Methyl Formamide:G lycerol Carbonate 20 : 80% | 17.8 | 24.16 | 17.1 | 78.38 | 3 |
| 10 | n-Methyl Formamide:Glycerol Carbonate 40 : 60% | 17.7 | 22.8 | 16.8 | 73.6 | 3 |
| 11 | n-Methyl Formamide:G lycerol Carbonate 60 : 40% | 17.6 | 21.5 | 16.5 | 68.7 | 3 |
| 12 | n-Methyl Formamide:G lycerol Carbonate 80 : 20% | 17.5 | 20.14 | 16.2 | 63.92 | 4 |
| 13 | Propylene Glycol Monomethyl Ether:Propylene Carbonate 55 : 45% | 17.6 | 11.6 | 8.2 | 92.4 | 6 |
| 14 | Propylene Glycol:Acetonitrile 20 : 80% | 15.6 | 16.5 | 9.1 | 57.1 | 4 |
| 15 | Propylene Glycol:Aceton itrile 40 : 60% | 15.9 | 15 | 12.2 | 61.2 | 1 |
| 16 | Propylene Glycol:Acetonitrile 60 : 40% | 16.2 | 13.4 | 15.2 | 65.4 | 1 |
| 17 | Propylene Glycol:Acetonitrile 80 : 20% | 16.5 | 11.92 | 18.26 | 69.54 | 1 |
| 18 | Propylene Carbonate: Propylene Glycol 65 : 35% | 18.9 | 15.3 | 10.1 | 81.2 | 6 |

| | | | | | | |
|---|---|---|---|---|---|---|
| *1 DMSO and NMP were removed from the data set as they are believed to outliers. | | | | | | |

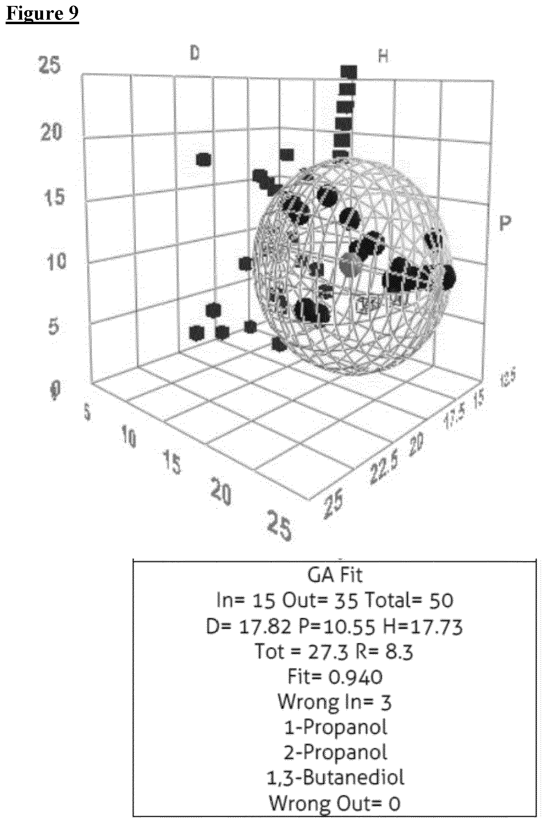

DMSO and NMP were removed from the data set as they are believed to be outliers, Propylene Glycol Monomethyl Ether:Propylene Carbonate 55 : 45% has a HSP that matches NMP and Propylene Carbonate:Propylene Glycol 65 : 35% matches the HSP of DMSO, Both of these blends did not dissolve which indicates that the result obtained in DMSO and NMP are outliers (see Figure 9).

Pal-GPKG has a moderate δD value, a moderate δP value and a moderate δH value. Dispersion forces provide the greatest contribution to the cohesive energy density of Pal-GPKG. The product has a radius of 8.3 units. The experimental data has a fit score of 0.94.

**Overall results summary**

| **Peptide** | **δD** | **δP** | **δH** | **R (Size of HSP Sphere)** | **Fit (Closer to 1 is best fit)** |
|---|---|---|---|---|---|
| Pal-GQPR (Matrixyl 3000) | - | - | - | - | - |
| Pal-QTAV | 17.61 | 10.66 | 10.95 | 6.0 | 1.000 |
| Idealift | 18.24 | 17.39 | 17.37 | 12.3 | 0.961 |
| Pal-EKGD | 17.31 | 13.92 | 17.26 | 10.3 | 1.000 |
| Pal-GHK (Matrixyl 3000) | 17.83 | 6.57 | 11.22 | 3.5 | 0.914 |
| Pal-LSVD | 18.84 | 12.04 | 14.82 | 6.8 | 0.930 |
| Pal-GPKG | 17.82 | 10.55 | 17.73 | 8.3 | 0.940 |

### 3. Optimisation of the solvents for matrikine compostions Formulating for Efficacy^{™} (FFE)

The HSP values from Example 1 were then entered into FFE software in order to predict the best solvent combination(s) to use for each matrikine (FFE is pre-loaded with some commercially available solvents and their HSPs). FFE was also used to predicts the amount of matrikine of the optimised composition that would be remains on the skin (on), diffuses into the stratum corneum (in) and goes beyond the stratum corneum and into the dermis (out).

The FFE tool was used to identify ingredients which could help deliver the biologically active peptides deeper into skin and therefore increase their efficacy.

FFE was used by first entering the HSP (Hansen Solubility Parameters), molecular volume and Melting point values or simplified molecular-input line-entry system (SMILES) of the peptide into the software (if not already preloaded).

Then, in the formulation part of the FFE software, various options were chosen to model the behaviour of the peptide with a range of preloaded ingredients or ingredients that we added via SMILES/HSP values.

As the peptides were predominately water soluble, optimisation focused on the water phase of the formulation. In short, optimisation involved looking at the materials on the database and making a judgement on the materials/levels that are appropriate for the formulation and comparing improvement of skin penetration to known and used formulations.

### Results

**Experiment 1 - Cosmetic materials to improve the Penetration of Pal-GHK**

| **System** | **Active On** | **Active In** | **Active Out** |
|---|---|---|---|
| Glycerin 81.51% | 0.00417 | 0.00003 | 0.00000 |
| Butylene Glycol 18.49% | | | |
| Baseline humectant water phase | | | |
| Glycerin 48.50% | 0.00408 | 0.00012 | 0.00000 |
| **PEG-7 Glyceryl Cocoate 33.01%** | | | |
| Butylene Glycol 18.49 | | | |
| Glycerin 48.50% | 0.00437 | 0.0005 | 0.00000 |
| Butylene Glycol 18.49% | | | |
| **Methyl Gluceth-20 33.01%** | | | |
| Glycerin 48.50% | 0.00389 | 0.00031 | 0.00000 |
| Butylene Glycol 18.49% | | | |
| **Bis-PEG-18 Methyl Ether Dimethyl** | | | |
| **Silane 33.01%** | | | |

The PEG-7 Glyceryl Cocoate, Methyl Glucerth-20 and bis-PEG-18 methyl ether dimethyl silane all increased theorical the amount of Pal GHK into the skin. Used at cosmetically acceptable levels without negativity effecting textural attributes.

**Experiment 2 - Understand effect of Methyl Gluceth-20 with Pal-LSVD and Pal-GPKG in a cosmetic water phase humectant system**

| **System** | **Peptide** | **Active On** | **Active In** | **Active Out** |
|---|---|---|---|---|
| Glycerin 43.55% | Pal-LSVD | 0.00496 | 0.00004 | 0.00000 |
| Butylene Glycol 56.45% | | | | |
| Baseline humectant water phase | | | | |
| | | | | |
| Glycerin 29.75% | Pal-LSVD | 0.00493 | 0.00007 | 0.00000 |
| Butylene Glycol 40.66% | | | | |
| **Methyl Gluceth-20 29.75%** | | | | |
| | Pal-GPKG | 0.00495 | 0.00005 | 0.00000 |
| Glycerin 43.55% | | | | |
| Butylene Glycol 56.45% | | | | |
| Baseline humectant water phase | | | | |
| Glycerin 29.75% | Pal-GPKG | 0.00493 | 0.00007 | 0.00000 |
| Butylene Glycol 40.66% | | | | |
| **Methyl Gluceth-20 29.75%** | | | | |

## Claims

1. A method of choosing a solvent for a matrikine, the method comprising:
(a) identifying the Hansen Solubility Parameter (HSP) sphere of
skin,
the matrikine, and
two or more test solvents for the matrikine; and
(b) choosing at least one of the two or more test solvents based on
the proximity of its HSP sphere to the HSP sphere of skin, and/or
the proximity of its HSP sphere to the HSP sphere of the matrikine,
wherein the matrikine has an amino acid sequence selected from the group consisting of:
(i) GPXG, (ii) LSXX, (iii) XXGD, (iv) QTAV, (v) GHK, and (vi) GQPR,
wherein for (i) G denotes the amino acid glycine and P denotes the amino acid proline (as per the internationally recognised single letter code for amino acids), and X denotes an amino acid selected from the group consisting of Lysine (K), Glutamic acid (E) and Serine (S) and mixtures thereof,
wherein for (ii) L denotes the amino acid Leucine and S denotes the amino acid Serine (as per the internationally recognised single letter code for amino acids), and X denotes an amino acid selected from the group consisting of Valine (V), Aspartic acid (D), Proline (P), Glycine (G) and mixtures thereof,
wherein for (iii) G denotes the amino acid Glycine and D denotes the amino acid Aspartic acid (as per the internationally recognised single letter code for amino acids), and X denotes an amino acid selected from the group consisting of Glutamic acid (E), Lysine (K), Leucine (L), Alanine (A), Isoleucine (I), Arginine (R) and mixtures thereof,
wherein for (iv) Q denotes the amino acid Glutamine, T denotes the amino acid Threonine, A denotes the amino acid Alanine, and V denotes the amino acid Valine,
wherein for (v), G denotes the amino acid Glycine, H denotes Histidine, and K denotes the amino acid Lysine (as per the internationally recognised single letter code for amino acids), and
wherein for (vi), G denotes the amino acid Glycine, Q denotes the amino acid Glutamine, and P denotes the amino acid Proline, and R denotes the amino acid Arginine (as per the internationally recognised single letter code for amino acids).

2. The method of claim 1, wherein identifying the HSP sphere of the matrikine comprises determining the HSP sphere of a composition comprising the matrikine, determining the HSP sphere of an excipient composition comprising the matrikine, or determining the HSP sphere of the pure matrikine.

3. The method of claim 1 or claim 2, wherein identifying the HSP sphere comprises using one or more different reference solvents with known HSP values to try and dissolve the matrikine, the two or more test solvents and skin.

4. The method of claim 3, wherein identifying the HSP sphere comprises ranking each of the one or more reference solvents based on their ability to dissolve skin, the matrikine and/or a test solvent by using the qualitative criteria of a solubility rank.

5. The method of claim 4, wherein the solubility rank uses the following qualitative criteria:
| **Solubility Rank** | **Precipitate** | **Turbidity** |
|---|---|---|
| 1 | None | None |
| 2 | None | Weak |
| 3 | None | Strong |
| 4 | Trace | Strong |
| 5 | Moderate | Weak |
| 6 | Complete | None |

6. The method of claim 5, wherein identifying the HSP sphere comprises using one or more, two or more, three or more, four or more, five or more, six or more, seven or more, eight or more, nine or more, 10 or more, 11 or more, 12 or more 13 or more, 14 or more, 15 or more, 16 or more, 17 or more, 18 or more, 19 or more, 20 or more, 25 or more, 30 or more, 35 or more, 40 or more, 45 or more, or 50 or more different reference solvents with a solubility rank of 3, 2 and 1; 2 and 1; or 1.

7. The method according to any one of the preceding claims, comprising choosing the at least one test solvent because its HSP sphere partially or completely overlaps with the HSP sphere of the matrikine.

8. The method according to any one of the preceding claims, comprising choosing a test solvent because the centre of its HSP sphere is closer to the centre of the HSP sphere of the matrikine than the centre of the HSP spheres of non-chosen solvent(s).

9. The method according to any one of the preceding claims, comprising choosing a test solvent because its HSP sphere partially or completely overlaps with the HSP sphere of skin.

10. The method according to any one of the preceding claims, comprising choosing a test solvent because the centre of its HSP sphere is closer to the centre of the HSP sphere of the skin than the HSP sphere(s) of non-chosen solvent(s).

11. The method according to any one of claims 1 to 6, comprising choosing and mixing two or more (non) solvents, or three or more (non)solvents from a list of four or more, five or more, six or more, seven or more, eight or more, nine or more, 10 or more, 11 or more, 12 or more 13 or more, 14 or more, 15 or more, 16 or more, 17 or more, 18 or more, 19 or more, 20 or more, 25 or more, 30 or more, 35 or more, 40 or more, 45 or more, or 50 or more (non)solvents to form a solvent mixture, wherein the chosen (non)solvents have HSP values at opposite ends of the HSP sphere of skin or the HSP sphere of the matrikine.

12. The method according to any one of the preceding claims, wherein the matrikine is or comprises Pal-GHK, or is or comprises Pal-GQPR.

13. A method of creating a composition comprising a matrikine, the method comprising choosing a solvent for a matrikine using the method according to any one of claims 1 to 12; and dissolving the matrikine in the chosen solvent(s).

14. A composition obtained or obtainable by the method of claim 13.

15. A method of delivering a composition of according to claim 14 to the skin of a subject., the method comprising contacting the with the skin of said subject.
